# EUROPEAN PATENT SPECIFICATION

(11) **EP 3 264 083 B1**
(45) Date of publication and mention of the grant of the patent: **25.09.2019**
(21) Application number: 16755607.5
(22) Date of filing: 25.02.2016
(51) Int. Cl.: G01N 33/53, G01N 33/543, G01N 33/92

(54) **L-FABP IMMUNOASSAY METHOD**
L-FAB-IMMUNOASSAY-VERFAHREN
PROCÉDÉ DE DOSAGE IMMUNOLOGIQUE DE L-FABP

(30) Priority: 25.02.2015 JP 2015034951
(43) Date of publication of application: 03.01.2018
(60) Divisional application: 19178394.3
(73) Proprietor: Sekisui Medical Co., Ltd., Tokyo 103-0027 (JP)
(72) Inventor: KOBAYASHI, Koji, Tokyo 103-0027 (JP); MATSUMOTO, Takuji, Tokyo 103-0027 (JP); YAMAMOTO, Mitsuaki, Tokyo 103-0027 (JP)
(74) Representative: Hoffmann Eitle
(86) International application number: PCT/JP2016/055576
(87) International publication number: WO 2016/136863

(56) References cited:
- EP-A1- 2 568 291
- WO-A1-2005/010528
- WO-A1-2007/074860
- JP-A- H11 242 026
- JP-A- H11 281 646
- US-A1- 2009 311 729
- US-A1- 2010 167 310
- US-A1- 2012 135 433
- US-B1- 7 592 148
- KENJIRO KIMURA: 'L-FABP no Rinshoteki Igi to Kongo no Tenbo' JAPANESE JOURNAL OF CLINICAL LABORATORY AUTOMATION vol. 39, no. 4, 2014, page 433, XP009505461
- NOBUHIRO SATO: 'Nyo-chu L-FABP no Sokuji Sokutei o Kano ni suru Shinki Latex-ho Shiyaku Norudia L-FABP no Kisoteki Seino Hyoka' JAPANESE JOURNAL OF CLINICAL LABORATORY AUTOMATION vol. 40, no. 4, 01 September 2015, page 523 269, XP009505460

## Description

### TECHNICAL FIELD

The present invention relates to an immunological measurement method for L-FABP using an anti-L-FABP antibody.

### BACKGROUND ART

Fatty acid binding proteins (FABPs) are a group of proteins present in the cytosol and having a molecular weight of about 14 kilodalton and an ability to bind to a fatty acid and include at least seven known molecular species such as a liver type (L-FABP), an intestinal type (I-FABP), a heart type (H-FABP), a brain type (B-FABP), a skin type (C-FABP/E-FABP), an adipocyte type (aP2), and a peripheral nerve cell type (myelin P2), and these are thought to be a family evolved from a common ancestral gene. While each type of FABP shows a specific tissue distribution, the name indicates the tissue in which the type was first found, and does not necessarily means that the type exists only in that tissue. For example, at least two types of FABP, the liver type (L-FABP) and the heart type (H-FABP) are expressed in the human kidney tissue and, among them, L-FABP is distributed in the proximal tubule, while H-FABP is mainly distributed in the distal tubule (Maatman et al., Biochemical Journal, vol.288, pp.285-290, 1992; Maatman et al., Biochemical Journal, vol.273, pp.759-766, 1991).

Patent Document 1 discloses a kidney disease examination method characterized by focusing on a relationship between the expression of L-FABP in the kidney tissue and the prognosis of kidney disease and detecting a fatty acid binding protein derived from the kidney tissue present in the test sample. More specifically, in a described example, urine collected from a renal disease patient was used as a sample to measure an amount of L-FABP leaked into the urine with sandwich ELISA using an anti-mouse L-FABP polyclonal antibody. However, detailed measurement conditions (conditions of sample processing and an antigen-antibody reaction), detection sensitivity, measurement values, etc. are not described.

In Patent Document 2, a prognostication method for sepsis or multiple organ failure is disclosed in which L-FABP is detected by ELISA using a specific antibody to compare the detected values in collected urine samples before and after treatment with a redox reagent, according to the findings that the immunoreactivity of urinary L-FABP was enhanced by treating urine with a redox reagent such as hemin (chloro[3,7,12,17-tetramethyl-8,13-divinylporphyrin-2,18-dipropanoato(2-)]iron (III), also referred to as a chloro(porphyrinato) iron (III) complex) and that the prognosis of patients with sepsis or multiple organ failure was poorer when the degree of enhancement (fold induction) was larger.

Additionally, Patent Document 3 discloses a method in which one or two compounds from a reducing agent (glutathione, cysteine, penicillamine, etc.), a chaotropic reagent (urea, guanidine, etc.), and a surfactant (sodium n-dodecylbenzenesulfonate etc.) are added as a denaturing agent to a urine sample for pretreating the urine sample with these compounds so as to improve the sensitivity of immunoassay, i.e., the measurement sensitivity to a urinary protein that is an analyte, and L-FABP is described as an example of the urinary protein. However, it is only described as a specific example of a method of measuring a urinary protein that megalin was treated with a denaturing agent for detection with sandwich ELISA using an anti-human megalin LBD1 monoclonal antibody recognizing two different epitopes, and no specific description about the detection of L-FABP is included.

On the other hand, Patent Document 4 discloses an examination method for nephropathy and an examination kit thereof characterized by using a probe labeled with quantum dots and detecting L-FABP derived from the kidney tissue present in a test sample. A specific example described in the document is an immunochromatography using a probe labeled with quantum dots, and a urine sample not subjected to an operation such as dilution was dropped and developed for immunochromatography, and a captured amount of the complex between L-FABP and the quantum dot-labeled monoclonal antibody was observed with the naked eye through red fluorescence when excitation light (wavelength: 350 nm) was applied.

Patent Document 5 discloses a reagent for measuring agglutination by using a specific amine compound, whereby aggregation based on a specific reaction can be accelerated without causing spontaneous agglutination of carrier particles, and measurement method.

Patent Document 6 provides a quick and convenient determination method and a determination reagent for acute enterocolitis, which can determine acute enterocolitis quantitatively and objectively.

Patent Document 7 discloses a prognosis diagnosis method which can diagnose the prognosis of a patient suffering from sepsis or sepsis-related multiple organ failure in a simple manner and with high accuracy and a prognosis diagnosis kit for use in the prognosis diagnosis method.

Patent Document 8 discloses a method for examining kidney disease, which comprises detecting fatty acid binding protein derived from kidney tissues, which is present in specimen collected from mammal excluding rodents.

Patent Document 9 provides means and methods for diagnosing acute kidney injury associated after an acute event or after a surgical intervention based on the biomarker L-FABP.

### CITATION LIST

### PATENT LITERATURE

Patent Document 1: Japanese Laid-Open Patent Publication No. HI 1-242026
Patent Document 2: Japanese Laid-Open Patent Publication No. 2011-22000
Patent Document 3: Japanese Laid-Open Patent Publication No. 2014-85208
Patent Document 4: WO 2009/081680
Patent Document 5: US 2010/167310 A1
Patent Document 6: US 2009/311729 A1
Patent Document 7: US 2012/135433 A1
Patent Document 8: US 7,592,148 B1
Patent Document 9: EP 2 568 291 A1

### SUMMARY OF INVENTION

### TECHNICAL PROBLEM

The present inventors attempted to detect L-FABP by a latex turbidimetric immunoassay (hereinafter sometimes referred to as LTLA) so as to develop a quicker and
easier method of measuring L-FABP. When L-FABP was measured with an LTIA reagent having a general construction including a buffer solution as a first reagent and a buffer solution containing anti-L-FABP antibody-immobilized latex particles as a second reagent, a desired sensitivity was not acquired and, in measurement of urinal L-FABP, a desired performance was also not acquired in terms of correlation with an ELISA-based in vitro diagnostic product (Renapro (registered trademark) L-FABP test TMB (manufactured by Cmic Holdings)).

A problem to be solved by the present invention is to provide an immunological measurement method for L-FABP in a sample using an anti-L-FABP antibody having a detection sensitivity equal to or greater than and having a favorable correlation with that of an existing ELISA-based in vitro diagnostic product.

### SOLUTION TO PROBLEM

[1] A method of detecting L-FABP (liver-type fatty acid binding protein) in a sample with an anti-L-FABP antibody by a particle agglutination measurement method, comprising the step of: bringing an anti-L-FABP antibody and a compound having a partial structure of NH₂-C=N- and a cyclic structure in a molecule into contact with a sample suspected of containing L-FABP, wherein
   the step of bringing the anti-L-FABP antibody and the compound having a partial structure of NH₂-C=N- and a cyclic structure in a molecule into contact with the sample suspected of containing L-FABP is performed in the order in which
   a step of bringing the compound into contact with the sample suspected of containing L-FABP is followed by a step of bringing the compound into contact with the anti-L-FABP antibody;
   wherein the compound having a partial structure of NH₂-C=N- and a cyclic structure in a molecule is one or two or more selected from a compound represented by Formula (1) or salt or ester thereof and a compound represented by Formula (2) or salt thereof:
      the compound of Formula (1) 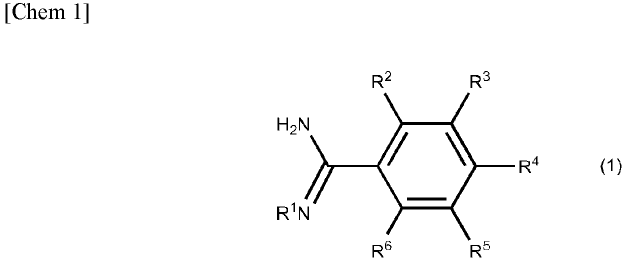 [in Formula (1), R¹ is a hydrogen atom, a hydroxyl group, or an alkyl group having the carbon number of 1, 2, or 3 that may be branched, and R² to R⁶ each independently represent a hydrogen atom, a halogen atom, an alkyl group having the carbon number of 1, 2, or 3 that may be branched, a hydroxyl group, a carboxy group, an amino group, or -SR⁷ (R⁷ represents a hydrogen atom, a hydroxyl group, or an alkyl group having the carbon number of 1, 2, or 3 that may be branched and, when a plurality of R⁷s are present, R⁷s may be the same groups as or different groups from each other)] or salt or ester thereof; and
      the compound of Formula (2) 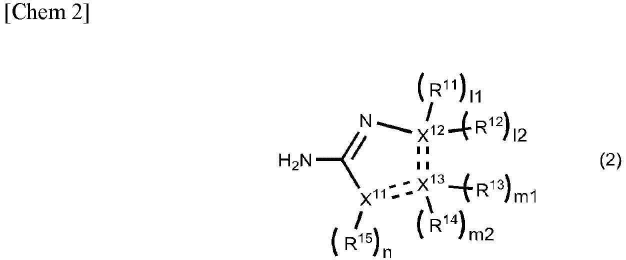
         [in Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together, R¹⁵ represents a hydrogen atom, a halogen atom, or an alkyl group having the carbon number of 1, 2, or 3 that may be branched,
         X¹¹ is a nitrogen atom or a sulfur atom,
         X¹² and X¹³ are each independently a carbon atom or a nitrogen atom, and
         l1, l2, m1, m2, and n are each independently 0 or 1,
         a double broken line between X¹¹ and X¹³ and a double broken line between X¹² and X¹³ are each independently a single bond or a double bond, wherein
         the values of l1, l2, m1, m2, and n as well as the bonds of the double broken line between X¹¹ and X¹³ and the double broken line between X¹² and X¹³ indicate values and bonds appropriately determined depending on the valences of X¹¹ to X¹³], or salt thereof.
[2] The method according to [1], wherein the compound represented by Formula (2) or salt thereof is one or two or more selected from the following compound or salt thereof:
   the compound of Formula (2) 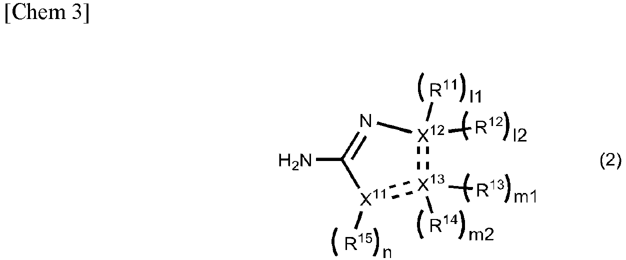
   [In Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together, R¹⁵ represents a hydrogen atom, a halogen atom, or an alkyl group having the carbon number of 1, 2, or 3 that may be branched], wherein
   in a combination of X¹¹ to X¹³, l1+l2, m1+m2, n (l1, l2, m1, m2, and n each independently represent 0 or 1), and double broken lines,
      (a) X¹¹ is a sulfur atom, X¹² and X¹³ are carbon atoms, l1+l2 is 2, m1+m2 is 2, n is 0, and the double broken lines between X¹¹ and X¹³ and between X¹² and X¹³ are single bonds,
      (b) X¹¹ is a sulfur atom, X¹² and X¹³ are carbon atoms, l1+l2 is 1, ml+m2 is 1, n is 0, the double broken line between X¹¹ and X¹³ is a single bond, and the double broken line between X¹² and X¹³ is a double bond,
      (c) X¹¹ is a nitrogen atom, X¹² and X¹³ are carbon atoms, l1+l2 is 2, m1+m2 is 2, n is 1, and the double broken lines between X¹¹ and X¹³ and between X¹² and X¹³ are single bonds,
      (d) X¹¹ is a nitrogen atom, X¹² and X¹³ are carbon atoms, l1+l2 is 1, m1+m2 is 1, n is 1, the double broken line between X¹¹ and X¹³ is a single bond, and the double broken line between X¹² and X¹³ is a double bond,
      (e) X¹¹ and X¹² are nitrogen atoms, X¹³ is a carbon atom, l1+l2 is 1, m1+m2 is 1, n is 0, the double broken line between X¹¹ and X¹³ is a double bond, and the double broken line between X¹² and X¹³ is a single bond, or
      (f) X¹¹, X¹², and X¹³ are nitrogen atoms, l1+l2 is 0, m1+m2 is 0, n is 1, the double broken line between X¹¹ and X¹³ is a single bond, and the double broken line between X¹² and X¹³ is a double bond, or salt thereof.
[3] The method according to any one of [1] to [2], wherein at the step of bringing the anti-L-FABP antibody and the compound having a partial structure of NH₂-C=N- and a cyclic structure in a molecule into contact with a sample suspected of containing L-FABP, the concentration of the compound is 300 mmol/L to 500 mmol/L.
[4] The method according to any one of [1] to [3], wherein the anti-L-FABP antibody is immobilized on an insoluble carrier, and the insoluble carrier is a latex particle, or a metal colloid particle.
[5] The method according to any one of [1] to [4], wherein the anti-L-FABP antibody is two or more monoclonal antibodies having recognition sites different from each other.
[6] The method according to [5], wherein the two or more monoclonal antibodies having recognition sites different from each other are respectively immobilized on latex particles, and wherein L-FABP is detected by a latex turbidimetric immunoassay.
[7] The method according to any one of [1] to [6], wherein the sample is urine, whole blood, serum, or plasma.
[8] A method of improving measurement sensitivity in a method of detecting L-FABP in a sample with an anti-L-FABP antibody by a particle agglutination measurement method, comprising the step of: bringing an anti-L-FABP antibody and a compound having a partial structure of NH₂-C=N- and a cyclic structure in a molecule into contact with a sample suspected of containing L-FABP, wherein
   the step of bringing the anti-L-FABP antibody and the compound having a partial structure of NH₂-C=N- and a cyclic structure in a molecule into contact with the sample suspected of containing L-FABP is performed in the order in which
   a step of bringing the compound into contact with the sample suspected of containing L-FABP is followed by a step of bringing the compound into contact with the anti-L-FABP antibody;
   wherein the compound having a partial structure of NH₂-C=N- and a cyclic structure in a molecule is one or two or more selected from a compound represented by Formula (1) or salt or ester thereof and a compound represented by Formula (2) or salt thereof:
      the compound of Formula (1) 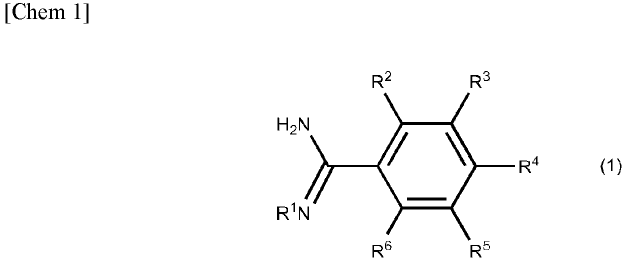 [in Formula (1), R¹ is a hydrogen atom, a hydroxyl group, or an alkyl group having the carbon number of 1, 2, or 3 that may be branched, and R² to R⁶ each independently represent a hydrogen atom, a halogen atom, an alkyl group having the carbon number of 1, 2, or 3 that may be branched, a hydroxyl group, a carboxy group, an amino group, or -SR⁷ (R⁷ represents a hydrogen atom, a hydroxyl group, or an alkyl group having the carbon number of 1, 2, or 3 that may be branched and, when a plurality of R⁷s are present, R⁷s may be the same groups as or different groups from each other)] or salt or ester thereof; and
      the compound of Formula (2) 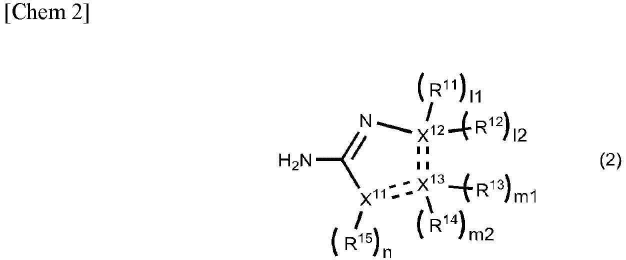
         [in Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together, R¹⁵ represents a hydrogen atom, a halogen atom, or an alkyl group having the carbon number of 1, 2, or 3 that may be branched,
         X¹¹ is a nitrogen atom or a sulfur atom,
         X¹² and X¹³ are each independently a carbon atom or a nitrogen atom, and
         l1, l2, m1, m2, and n are each independently 0 or 1,
         a double broken line between X¹¹ and X¹³ and a double broken line between X¹² and X¹³ are each independently a single bond or a double bond, wherein
         the values of l1, 12, m1, m2, and n as well as the bonds of the double broken line between X¹¹ and X¹³ and the double broken line between X¹² and X¹³ indicate values and bonds appropriately determined depending on the valences of X¹¹ to X¹³], or salt thereof.

### ADVANTAGEOUS EFFECTS OF INVENTION

According to the present invention, by bringing a compound according to Formula (1) or Formula (2) into contact with L-FABP in a sample, L-FABP in the sample can quickly and easily be detected with an immunological measurement method.

### BRIEF DESCRIPTION OF DRAWINGS

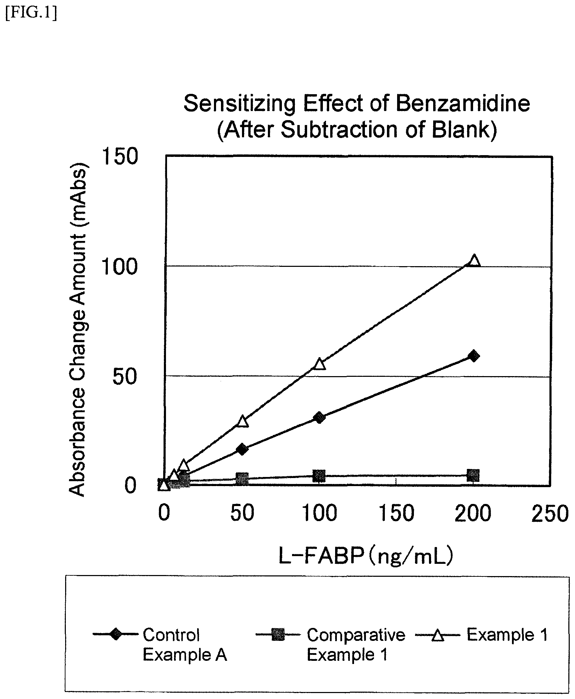

Fig. 1 is a diagram of a result of comparison of effects of a compound of Formula (1) (benzamidine hydrochloride) and a pretreatment liquid component of a reference method in LTIA.

### DESCRIPTION OF EMBODIMENTS

### (Sample)

Examples of the sample used in the present invention include urine, blood (whole blood, plasma, or serum), kidney tissue, extract from kidney tissue, etc. Among them, urine is a particularly preferred sample. Any samples are usable as long as the samples are suspected of containing L-FABP, including a sample derived from a healthy subject, a sample derived from a patient, a sample derived from a person suspected of having a disease, etc.

### (Anti-L-FABP Antibody)

For the anti-L-FABP antibody used in the present invention, native L-FABP purified from organs, cells, body fluid, etc. can be prepared as an immunogen (antigen). L-FABP is mainly distributed in the liver or the kidney and therefore can be purified and isolated from these organs etc. Additionally, it is known that L-FABP is highly homologous among humans, mice, pigs, cows, and rats and has a homology of 90% or more at the amino acid level and, therefore, for example, mouse L-FABP can be used as an antigen for acquiring an antibody binding to human L-FABP.

Purification of native L-FABP can be performed in accordance with the method described in Kelvin et al. (J. Biol. Chem., vol.263, pp.15762-15768, 1988) etc. In particular, after homogenizing an excised organ, a cytoplasmic fraction acquired by ultracentrifugation is fractionated by gel filtration, anion exchange chromatography, etc., and the fraction containing L-FABP is selected by using a molecular weight or fatty acid binding activity as an index, isolated, and purified. The selected fraction is subjected to SDS-polyacrylamide electrophoresis to confirm that the purified protein forms a single band and is further purified if needed. For the purified protein, the amino-acid composition and the N-terminal amino-acid sequence can be determined and compared with the reported composition and sequence so as to confirm that the protein is the intended molecular species.

L-FABP used as an antigen may be a recombinant protein produced by a genetic engineering technique. Since the amino acid sequence and the gene sequence of L-FABP are already reported (Veerkamp and Maatman, Prog. Lipid Res., vol.34, pp. 17-52, 1995), for example, a primer can be designed based on these sequences for cloning of cDNA from an appropriate cDNA library etc. by a PCR (polymerase chain reaction) method. This cDNA can be used for performing gene recombination so as to prepare recombinant L-FABP. Additionally, a fragment of L-FABP or a synthetic peptide etc. having a partial sequence thereof can be bound to a carrier macromolecular substance (BSA, hemocyanin, etc.) as needed and used as the antigen.

An antibody specifically binding to L-FABP may be any of antisera, polyclonal antibodies, monoclonal antibodies, etc.

The antibody preferably has a high specificity and, for example, in the case of the anti-L-FABP antibody, desirably, the antibody is substantially not cross-reactive with H-FABP. To acquire an antibody with a higher specificity, a highly purified and highly pure antigen is desirably used. When an antibody is prepared, a warm-blooded animal other than human is immunized by inoculating the purified antigen prepared as described above. Examples of the warm-blooded animal to be immunized other than human include mammals (rabbits, sheep, rats, mice, guinea pigs, horses, pigs, etc.) and birds (chickens, ducks, geese etc.). In the case of rabbits, for example, about 100 µg to 1 mg of the antigen emulsified in about 1 mL of saline and Freund's complete adjuvant is inoculated subcutaneously in the dorsum or the palm of a hind foot and, from the second time, the adjuvant is replaced with Freund's incomplete adjuvant, the antigen is inoculated three to eight times at intervals of two to four weeks for immunization, and the antibody is produced about 7 to 12 days after the final inoculation and used. In the case of mice, 10 to 30 µg/animal of the antigen is usually inoculated subcutaneously, intraperitoneally, or intravenously three to eight times at intervals of about two weeks for immunization, so as to use the antibody produced about two to four days after the final inoculation.

The polyclonal antibodies can be prepared by collecting blood from the animal immunized as described above, separating serum (antiserum), and recovering an Ig fraction from the acquired antiserum. For example, polygonal IgG can be acquired by recovering an IgG fraction from the antiserum by affinity chromatography using a Protein G column etc.

The monoclonal antibodies are produced by a hybridoma acquired by fusing an antibody-producing cell collected from an immunized animal with an immortalized cell. Mice and rats are preferably used as immunized animals for the monoclonal antibodies. The hybridoma can be produced in accordance with the method of Kohler and Milstein (Kohler and Milstein, Nature, vol.256, pp.495-887, 1975) as follows. Antibody-producing cells (such as splenocytes or lymph node cells) from the animal immunized as described above are collected and fused with appropriate immortalized cells. For example, cell lines of myeloma cells (NSI-Ag 4/1, Sp2/O-Ag14 etc.) are preferably used as the immortalized cells. The myeloma cells are preferably non-secretors not producing antibodies or immunoglobulin H or L chains by themselves. The myeloma cells preferably have a selection marker so that unfused myeloma cells and fused hybridomas may be screened in a selection medium. For example, for the selection marker, cell lines having 8-azaguanine resistance (hypoxanthine-guanine-phosphoribosyltransferase deficiency), thymidine kinase deficiency, etc. are often used. The cell fusion is performed by adding an appropriate fusion promoter such as polyethylene glycol. The cell fusion is preferably performed at a ratio of about 10 antibody-producing cells per immortalized cell, and can preferably be performed at a cell density of about 10⁶ cells/mL of the antibody-producing cells.

The cells subjected to the fusion treatment are appropriately diluted and then cultured in a selection medium for one to two weeks. For example, when myeloma cells resistant to 8-azaguanine are used, the unfused myeloma cells cultured in the HAT (hypoxanthine, aminopterin, thymidine) medium die, and the unfused antibody-producing cells cultured in the HAT (hypoxanthine, aminopterin, thymidine) medium also die because of limited division cycle; however, only the fused cells can continue to undergo division and survive in the selection media. After culturing in the selection medium, the supernatant is subjected to, for example, ELISA with an antigen immobilized on a solid phase, so as to detect the presence/absence of the intended antibody, and cloning can be performed by a limiting dilution method to select a hybridoma producing a monoclonal antibody recognizing the intended antigen. The hybridoma is selected that produces the monoclonal antibody having desired properties such as antibody titer, antibody class, subclass, affinity for antigen, specificity, epitope, etc. IgG is generally preferable as the class of monoclonal antibody.

The monoclonal-antibody-producing hybridoma is intraperitoneally implanted in an animal of the same species as the animal used for immunization and, after elapse of a certain period, the ascites can be collected from the animal to isolate the intended monoclonal antibody. Alternatively, the hybridoma can be cultured in an appropriate animal cell culture medium, and the monoclonal antibody can be isolated from the culture solution. Once the object hybridoma is acquired, the gene encoding the monoclonal antibody can be acquired from the hybridoma to express and produce the intended monoclonal antibody in an appropriate host (e.g., silkworm, etc.) by a common gene recombination technique. Separation and purification of the antibody can be performed in accordance with, for example, a usual purification method combining ammonium sulfate precipitation, gel chromatography, ion exchange chromatography, affinity chromatography, etc. as needed.

The anti-L-FABP antibody used in the present invention may be a known antibody or an antibody to be developed in the future. Although not particularly limited, usable commercially available anti-L-FABP antibodies include C-4 (catalog No. sc-374537), F-9 (catalog No. sc-271591) of Santa Cruz Biotechnology, 328607 (catalog No. MAB2964) of R&D systems, L2B10 (Catalog No. HA 2049-IA) of Hycult biotech, 2G4 (Catalog No. LS-B3001) of Lifespan Biosciences, etc. These antibodies bind to polypeptides of the internal region, the N-terminal region, etc. of the human-derived L-FABP protein and, even when the antibodies bind to the internal region of the L-FABP molecule, L-FABP can be detected with higher sensitively and higher specificity by using a compound having a partial structure of NH₂-C=N- and a cyclic structure in a molecule of the present disclosure.

"Antibody" in the present invention includes not only intact immunoglobulin molecules but also antibody fragments or antibody derivatives having an antigen binding ability known in the art, such as Fab, Fab'2, CDR, a humanized antibody, a multifunctional antibody, and a single chain antibody (ScFv).

### (Detection)

The method of detecting L-FABP using an anti-L-FABP antibody of the present disclosure is an immunological measurement method. More specifically, a particle immunoagglutination measurement method such as a latex turbidimetric immunoassay (LTIA), ELISA, a chemiluminescence detection method, and immunochromatography (lateral-flow type, flow-through type) are discussed. Among them, a particle immunoagglutination measurement method not including a step for B/F separation (homogeneous immunoassay method) is more preferable.

It is noted that when LTIA is described as a measurement method in this description, the detection method thereof may be achieved by using any of known detection methods such as measurement of change in transmitted light (absorbance), measurement of change in scattered light, and measurement of change in particle in the description.

Additionally, it may easily be understood by those skilled in the art that the present disclosure is applicable in an immunohistological staining method, an electrophoresis method (western blotting etc.), a dot blotting method, etc., as long as the immunological measurement using an anti-L-FABP antibody is performed by using a sample contacted with a compound having a partial structure of NH₂-C=N- and a cyclic structure in a molecule of the present disclosure.

Furthermore, the term "detection" or "measurement" must be construed in the broadest sense including the proof of the presence and/or the quantification of L-FABP, and must not be construed as a limitation.

### (Insoluble Carrier)

An insoluble carrier used in the present invention can be an insoluble carrier made of a polymeric base material such as polystyrene resin, an inorganic base material such as glass, a polysaccharide base material such as cellulose and agarose, etc. An insoluble carrier of the present disclosure is not particularly limited in terms of the shape thereof, and any shapes can be selected in accordance with the measurement method to be adopted, including a bead or particle shape (e.g., latex particles, metal colloid particles), a plate or sheet shape (e.g., a porous membrane, an immunoplate), a tubular shape (e.g., a test tube), etc.

Examples of the particles include latex particles mainly composed of polystyrene generally used in the particle immunoagglutination measurement method as well as particles containing a styrene-butadiene copolymer, a (meth)acrylic acid ester polymer, etc. as a base material. Particles made of metal colloid, gelatin, liposome, microcapsule, silica, alumina, carbon black, metallic compound, metal, ceramics, or magnetic material are also usable. For the carrier particles used in the present invention, one and the same kind of material or two or more kinds of materials can be used.

The particle diameter of the carrier particles is preferably 0.15 to 0.45 µm, more preferably 0.2 to 0.4 µm. Two or more kinds of the carrier particles different in average particle diameter can be combined and used.

The porous membrane can be a known membrane and can be made of any material. Examples of the material of the porous membrane include, but not limited to, polyethylene, polyethylene terephthalate, nylons, glass, polysaccharides such as cellulose and cellulose derivatives, ceramics, etc. Specifically, the examples include glass fiber filter paper, cellulose filter paper, etc. sold by Millipore, Toyo Roshi, Whatman, etc.

The plate (immunoplate) can be a known plate and can be made of any material. Examples of the material of the plate include, but not limited to, synthetic polymeric compounds such as vinyl chloride, polyethylene, polystyrene, polypropylene, and polyolefin elastomer, as well as glass etc.

### (Immobilization of Antibody to Insoluble Carrier)

A method for immobilizing the anti-L-FABP antibody on the insoluble carrier is not particularly limited, and any known method can be used.

If the anti-L-FABP antibody is immobilized on particles, this is achieved by using, for example, a physical adsorption method using physical adsorption caused by mixing particles and the antibody, or a chemical binding method using a coupling agent such as carbodiimide to chemically bind a carboxy group or an amino group on the particle surface to the antibody molecule. The antibody molecules may be immobilized on the particles via spacer molecules. Furthermore, after binding the antibody to another protein such as albumin by using the chemical binding method, the protein may physically or chemically be immobilized on the particles.

If the anti-L-FABP antibody is immobilized on the porous membrane, the antibody can be immobilized by, for example, applying a certain amount of a solution containing the antibody into a shape of a line, a dot, a specific symbol such as + to the porous membrane.

In this description, the "insoluble carrier" is referred to as a "solid phase", and allowing, or a state of allowing, the insoluble carrier to physically or chemically support an antigen or an antibody is referred to as "immobilization", "immobilized", "solid-phased", "sensitization", or "adsorption" in some cases.

### (Labeled Antibody)

Examples of a labeling substance for labeling the antibody include, for example, an enzyme, a fluorescent substance, a chemiluminescent substance, biotin, avidin, a radioactive isotope, gold colloid particles, or colored latex particles. A method of binding the labeling substance and the antibody can be methods such as a glutaraldehyde method, a maleimide method, a pyridyl disulfide method, or a periodic acid method available to those skilled in the art. Both the labeling substance and the binding method are not limited to those described above and a known method can be used.

With regard to detection of a label, for example, when an enzyme such as peroxidase or alkaline phosphatase is used as the labeling substance, the enzyme activity can be measured by using a specific substrate of the enzyme (e.g., 1,2-phenylenediamine or 3,3',5,5'-tetramethylbenzidine when the enzyme is horseradish peroxidase, or p-nitrophenyl phosphate in the case of alkaline phosphatase) and, when biotin is used as the labeling substance, avidin labeled at least with a labeling substance other than biotin is typically reacted therewith.

### (Compound Having Partial Structure of NH₂-C=N- and Cyclic Structure in Molecule)

A compound having a partial structure of NH₂-C=N- and a cyclic structure in a molecule of the present invention is compounds represented by Formula (1) and Formula (2) (sometime referred to as a "compound of Formula (1) and a "compound of Formula (2)" in the description).

The compound of Formula (1) is a compound of 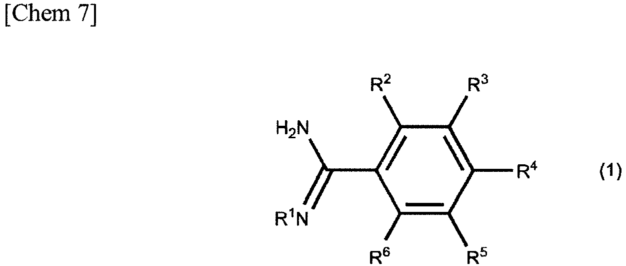 [in Formula (1), R¹ is a hydrogen atom, a hydroxyl group, or an alkyl group having the carbon number of 1, 2, or 3 that may be branched, and R² to R⁶ each independently represent a hydrogen atom, a halogen atom, an alkyl group having the carbon number of 1, 2, or 3 that may be branched, a hydroxyl group, a carboxy group, an amino group, or -SR⁷ (R⁷ represents a hydrogen atom, a hydroxyl group, or an alkyl group having the carbon number of 1, 2, or 3 that may be branched and, when a plurality of R⁷s are present, R⁷s may be the same groups as or different groups from each other)] or salt or ester thereof.

The compound of Formula (2) is a compound of 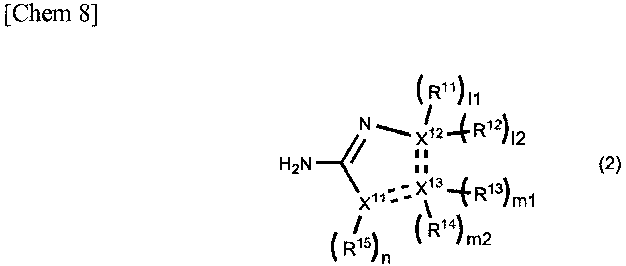
[in Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together, R¹⁵ represents a hydrogen atom, a halogen atom, or an alkyl group having the carbon number of 1, 2, or 3 that may be branched,
X¹¹ is a nitrogen atom or a sulfur atom,
X¹² and X¹³ are each independently a carbon atom or a nitrogen atom, and
11,12, m1, m2, and n are each independently 0 or 1,
a double broken line between X¹¹ and X¹³ and a double broken line between X¹² and X¹³ are each independently a single bond or a double bond, wherein
the values of l1, l2, m1, m2, and n as well as the bonds of the double broken line between X¹¹ and X¹³ and the double broken line between X¹² and X¹³ indicate values and bonds appropriately determined depending on the valences of X¹¹ to X¹³], or salt thereof.

Additionally, the compound of Formula (2) is a compound of 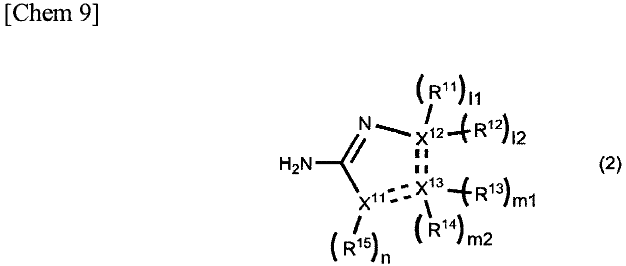
[In Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together, R¹⁵ represents a hydrogen atom, a halogen atom, or an alkyl group having the carbon number of 1, 2, or 3 that may be branched], wherein
in a combination of X¹¹ to X¹³, l1+l2, m1+m2, n (l1,l2, m1, m2, and n each independently represent 0 or 1), and double broken lines,
   (a) X¹¹ is a sulfur atom, X¹² and X¹³ are carbon atoms, l1+l2 is 2, m1+m2 is 2, n is 0, and the double broken lines between X¹¹ and X¹³ and between X¹² and X¹³ are single bonds,
   (b) X¹¹ is a sulfur atom, X¹² and X¹³ are carbon atoms, l1+l2 is 1, ml+m2 is 1, n is 0, the double broken line between X¹¹ and X¹³ is a single bond, and the double broken line between X¹² and X¹³ is a double bond,
   (c) X¹¹ is a nitrogen atom, X¹² and X¹³ are carbon atoms, l1+l2 is 2, m1+m2 is 2, n is 1, and the double broken lines between X¹¹ and X¹³ and between X¹² and X¹³ are single bonds,
   (d) X¹¹ is a nitrogen atom, X¹² and X¹³ are carbon atoms, l1+l2 is 1, m1+m2 is 1, n is 1, the double broken line between X¹¹ and X¹³ is a single bond, and the double broken line between X¹² and X¹³ is a double bond,
   (e) X¹¹ and X¹² are nitrogen atoms, X¹³ is a carbon atom, l1+l2 is 1, m1+m2 is 1, n is 0, the double broken line between X¹¹ and X¹³ is a double bond, and the double broken line between X¹² and X¹³ is a single bond, or
   (f) X¹¹, X¹², and X¹³ are nitrogen atoms, l1+l2 is 0, m1+m2 is 0, n is 1, the double broken line between X¹¹ and X¹³ is a single bond, and the double broken line between X¹² and X¹³ is a double bond, or salt thereof.

Examples of the compound represented by Formula (1) include a benzamidine derivative and examples of the compound represented by Formula (2) include an aminothiazole derivative, an aminotriazole derivative, an aminotetrazole derivative, and an aminoimidazole derivative. The salt of a compound according to Formula (1) or Formula (2) can be selected as needed from hydrochloride, sulfate, nitrate, hydrobromate, hydrofluoride, borofluoride, oxalate, lactate, adipate, tartrate, hydroiodide, toluenesulfonate, malonate, bicarbonate, etc. without particular limitation in considerations of the effect of the present invention as well as easiness of handling, availability, etc.

More specific examples of the compound of Formula (1) include benzamidine hydrochloride (CAS No. 1670-14-0), benzamidine hydrochloride hydrate (CAS No. 206752-36-5), 4-fluorobenzamidoxime (CAS No. 69113-32-2), and 4-chlorobenzamidine hydrochloride (CAS No. 14401-51-5). More specific examples of the compound of Formula (2) include aminothiazoline (CAS No. 1779-81-3), 2-amino-2-thiazoline hydrochloride (CAS No. 3882-98-2), pseudothiohydantoin (CAS No. 556-90-1), 2-amino-5-bromothiazole hydrobromide (CAS No. 61296-22-8), 2-amino-4,5-dimethylthiazole hydrobromide (CAS No. 7170-76-5), 2,4-diamino-5-phenylthiazole monohydrobromide (CAS No.:6020-54-8), and creatinine (CAS No. 60-27-5). Among them, benzamidine hydrochloride, 2-amino-2-thiazoline hydrochloride, and creatinine are particularly preferable. These compounds having a partial structure of NH₂-C=N- and a cyclic structure in a molecule may be used solely or may be used as a combination of two or more thereof.

A preferable range of the additive concentration of the compound according to Formula (1) or Formula (2) of the present invention may be 50 mmol/L to 1000 mmol/L, 50 mmol/L to 500 mmol/L, 50 mmol/L to 600 mmol/L, 100 mmol/L to 900 mmol/L, 200 mmol/L to 800 mmol/L, 300 mmol/L to 600 mmol/L, 300 mmol/L to 550 mmol/L, 300 mmol/L to 500 mmol/L, and 350 mmol/L to 450 mmol/L, and is preferably 50 mmol/L to 500 mmol/L, more preferably 300 mmol/L to 500 mmol/L. The optimum concentration of each of the compounds having a partial structure of NH₂-C=N- and a cyclic structure in a molecule to be used can experimentally be obtained as described in this description.

### (Method of Bringing Compound Having Partial Structure of NH₂-C=N- and Cyclic Structure in Molecule of the Present Disclosure into Contact with L-FABP in Sample and Method of bringing Anti-L-FABP Antibody into Contact with L-FABP in Sample)

A method of bringing the compound having a partial structure of NH₂-C=N- and a cyclic structure in a molecule of the present disclosure into contact with L-FABP in a sample can be, for example, a method of mixing a liquid reagent containing the compound having a partial structure of NH₂-C=N- and a cyclic structure in a molecule of the present disclosure with a sample. Another method of the present disclosure can be a method of supplying a sample to an insoluble carrier such as a porous membrane infiltrated with the compound having a partial structure of NH₂-C=N- and a cyclic structure in a molecule of the present disclosure so that the contact occurs.

Furthermore, L-FABP in the sample is brought into contact, by a known appropriate method, with an anti-L-FABP antibody immobilized on the insoluble carrier, after, or at the same time as, the contact with the compound having a partial structure of NH₂-C=N- and a cyclic structure in a molecule of the present disclosure.

Examples of the method of bringing the compound having a partial structure of NH₂-C=N- and a cyclic structure in a molecule of the present disclosure into contact with a sample include a method in which the compound is brought into contact as a diluent of a sample, an extraction solution of a sample, or a preservation solution, a development solution, etc. of a sample. The examples also include a method in which the compound is brought into contact with the sample together with an enzyme activity inhibitor, an anticoagulant, etc. as needed, in a container for sample collection such as a urine collection cup and a blood collection tube at the time of sample collection such as urine collection and blood collection.

### (Measurement Kit)

Constituents of a measurement kit described herein other than the compound having a partial structure of NH₂-C=N- and a cyclic structure in a molecule of the present disclosure are not particularly limited as long as L-FABP can immunologically be measured. The measurement kit will hereinafter be described by taking sandwich ELISA, immunochromatography, and LTIA as examples.

### <Sandwich ELISA>

In the case of the sandwich ELISA, the measurement kit includes at least (a) an insoluble carrier having an anti-L-FABP antibody of the present dislcosure immobilized thereon and (b) an antibody labeled with a labeling substance and having a property of reacting with L-FABP. In this case, the insoluble carrier is preferably a plate (immunoplate), and the labeling substance can appropriately be selected and used.

The antibody immobilized on the insoluble carrier captures L-FABP in the sample and forms a complex on the insoluble carrier. The antibody labeled with the labeling substance binds to the captured L-FABP and forms a sandwich with the complex described above. L-FABP in the sample can be measured by measuring an amount of the labeling substance with a method corresponding to the labeling substance. Specific methods such as a method of immobilizing the antibody on the insoluble carrier and a method of binding the antibody and the labeling substance can be achieved by using methods well known to those skilled in the art without particular limitation. Although either a homogeneous measurement method or a heterogeneous measurement method can be configured in the case of this configuration, the homogeneous measurement method is more preferable.

The compound having a partial structure of NH₂-C=N- and a cyclic structure in a molecule of the present disclosure can be added, for example, to a sample diluent or a solution for an antigen-antibody reaction and thereby can be brought into contact with L-FABP in the sample.

### <Immunochromatography>

Typical immunochromatography is configured by using a test strip equipped with "1. a sample-supply portion", "2. a portion of retaining a labeled antibody (a labeled antibody-retaining portion)", and "3. a portion of immobilizing an antibody for capturing a complex formed by the labeled antibody and the L-FABP antibody (a capture-antibody portion)" in the order in a direction of development of a solution containing a sample on a sheet-like insoluble carrier such as a porous membrane, such that the sample solution continuously moves due to capillarity. In the case of immunochromatography, the measurement kit at least includes the test strip as described above.

Specifically, when a predetermined amount of a sample containing L-FABP is added to the sample-supply portion, the sample enters the label-retaining portion due to capillarity, and L-FABP and the labeled antibody bind together to form a complex. When the complex developed through the membrane enters the capture-antibody portion, the complex is captured by the antibody (capture antibody) immobilized on the membrane to form a ternary complex of [the capture antibody]-[L-FABP]-[the labeled antibody]. The label can be detected by an arbitrary method (e.g., an agglutination image in the case of a label that can be made visible such as gold colloid particles, or a coloring reaction due to addition of a substrate in the case of an enzyme), so as to detect the presence of L-FABP.

For example, the compound having a partial structure of NH₂-C=N- and a cyclic structure in a molecule of the present disclosure can preliminarily be added to a sample diluent etc. or can preliminarily be contained in the sample-supply portion or the label-retaining portion and thereby can be brought into contact with L-FABP in the sample. For example, immunochromatography described in Patent Document 4 is a method capable of applying the present disclosure.

### <Latex Turbidimetric Immunoassay>

In the case of latex turbidimetric immunoassay, the measurement kit includes at least latex particles having an antibody immobilized thereon. For the antibody used in latex turbidimetric immunoassay, any combination of "two monoclonal antibodies having different recognition sites for antigens", "polyclonal antibodies", or "monoclonal antibody and polyclonal antibody" can be used. In this case, the latex particles are the insoluble carrier having an antibody immobilized thereon and the labeling substance at the same time.

The latex particles used for these measurement reagents can appropriately be selected in terms of particle diameter and material so as to acquire desired performance such as improved sensitivity. The latex particles may be any particles suitable for supporting the antibody. For example, the particles may contain polystyrene, a styrene-sulfonic acid (salt) copolymer, a styrene-methacrylic acid copolymer, an acrylonitrile-butadiene-styrene copolymer, a vinyl chloride- acrylic acid ester copolymer, a vinyl acetate-acrylic acid ester copolymer, etc., as a base material. Although the shape of the latex particles is not particularly limited, preferably, the average particle diameter is sufficiently large so that aggregates generated as a result of agglutination reaction between the antibody on the latex particle surfaces and L-FABP can be detected with the naked eye or optically. Particles made of material such as metal colloid, gelatin, liposome, microcapsule, silica, alumina, carbon black, metal compound, metal, ceramics, or magnetic material can be used instead of the latex particles.

A typical measurement kit for LTIA used in clinical examination is usually provided in a form of a first reagent and a second reagent. The latex particles having the antibody immobilized thereon described above can be contained in the first reagent or the second reagent. Although it is generally preferable that the latex particles having the antibody immobilized thereon be contained in the second reagent, the particles can be contained in the first reagent or in both the first and second reagents.

The compound having a partial structure of NH₂-C=N- and a cyclic structure in a molecule of the present disclosure is preferably contained in the first reagent. By mixing the first reagent containing the compound having a partial structure of NH₂-C=N- and a cyclic structure in a molecule of the present disclosure with the sample, L-FABP in the sample contacts with the compound having a partial structure of NH₂-C=N- and a cyclic structure in a molecule of the present disclosure.

In addition to those described above, the kit of the present disclosure appropriately includes a buffer component (buffer solution). The buffer solution usable in the present disclosure may be any commonly used buffer solutions including tris-hydrochloric acid, boric acid, phosphoric acid, acetic acid, citric acid, succinic acid, phthalic acid, glutaric acid, maleic acid, glycine, and salts thereof, and Good's buffers such as MES, Bis-Tris, ADA, PIPES, ACES, MOPSO, BES, MOPS, TES, and HEPES, for example.

The kit of the present disclosure also includes saccharides, proteins, etc. as needed for the purpose of improving measurement sensitivity and suppressing nonspecific reaction. Examples thereof include components promoting antigen-antibody reactions (polymeric compounds such as polyethylene glycol, polyvinyl pyrrolidone, phospholipid polymers etc.), proteins and peptides (albumin, casein, etc.), amino acids, sugars (sucrose, cyclodextrin, etc.), and preservatives (sodium azide, ProClin 300 etc.).

Although native L-FABP derived from tissues such as liver and kidney can be used as a standard substance (L-FABP standard substance) in sample measurement, the standard substance may be a recombinant protein produced by a genetic engineering technique. Since the amino acid sequence and the gene sequence of L-FABP has already been reported (Veerkamp and Maatman, Prog. Lipid Res., vol.34, pp. 17-52, 1995), for example, a primer can be designed based on these sequences for cloning of cDNA from an appropriate cDNA library etc. by a PCR (polymerase chain reaction) method. This can be used for preparing recombinant L-FABP by gene recombination techniques. For the standard substance, it is more preferable to use the recombinant protein with stable structure.

### EXAMPLES

Examples of the present invention will hereinafter be described to more specifically describe the present invention; however, the present invention is not limited thereto and can variously be applied without departing from the technical idea of the present invention.

For Examples 1 to 4, measurement was performed by using the following materials under the following conditions.

### (Anti-L-FABP Antibody-Immobilized Latex Particle Suspension)

### (1) Preparation of Clone L Antibody-Immobilized Latex Particle Suspension

To 13 mL of a 20 mmol/L Tris buffer solution (pH 8.5) containing 0.36 mg/mL of anti-L-FABP antibody Clone L (manufactured by CMIC HOLDINGS Co., Ltd.), 13 mL of a 1 % latex particle (manufactured by SEKISUI CHEMICAL CO., LTD.) suspension having an average particle diameter of 0.27 µm was added and stirred at 4 °C for two hours. This was followed by addition of 13 mL of a 20 mmol/L Tris buffer solution (pH 8.5) containing 0.5 % BSA and stirring at 4 °C for one hour. Subsequently, dialysis with a 5 mmol/L MOPS buffer solution (pH 7.0) was performed to acquire a Clone L antibody-immobilized latex particle suspension.

### (2) Preparation of Clone 2 Antibody-Immobilized Latex Particle Suspension

To 8 mL of a 20 mmol/L glycine buffer solution (pH 9.5) containing 0.54 mg/mL of anti-L-FABP antibody Clone 2 (manufactured by CMIC HOLDINGS Co., Ltd.), 8 mL of a 1 % latex particle (manufactured by SEKISUI CHEMICAL CO., LTD.) suspension having an average particle diameter of 0.25 µm was added and stirred at 4 °C for two hours. This was followed by addition of 8 mL of a 20 mmol/L glycine buffer solution (pH 9.5) containing 0.5 % BSA and stirring at 4 °C for one hour. Subsequently, dialysis with a 5 mmol/L MOPS buffer solution (pH 7.0) was performed to acquire a Clone 2 antibody-immobilized latex particle suspension.

### (L-FABP Standard Substance)

The L-FABP standard substance was acquired by gene recombination as described in Patent Document 1.

### (L-FABP Reference Measurement Method: Reference Method)

A pharmaceutical product for in-vitro diagnosis by ELISA (Renapro (registered trademark) L-FABP test TMB) was used for a reference method.

### (Control First Reagent: Also Serving as Standard Substance Diluent)

100 mmol/L phosphate buffer solution (pH 7.0)
300 mmol/L NaCl
0.2 % BSA
0.4 % Lipidure-BL103

### (Second Reagent)

5 mmol/L MOPS buffer solution (pH 7.0)
2.5 Abs/mL Clone L antibody-immobilized latex particle suspension^{(*)}
2.5 Abs/mL Clone 2 antibody-immobilized latex particle suspension^{(*)}
^{(*)} Abs denotes the absorbance at 280 nm.

### (Standard Solution)

The L-FABP standard substance was adjusted to a desired concentration by using the standard substance diluent and was used as a standard solution.

### (Freeze-Thawed Urine)

Partial urine frozen and stored at -30 °C after collection was thawed only once and used for measurement.

### (Healthy Serum)

Serum was acquired by collecting blood from 50 volunteers having no previous history causing a concern over variation in L-FABP in the body and actually having a urinary L-FABP concentration within a reference range and was pooled as healthy serum. The healthy serum was used on the day of blood collection and pooling. For reference, the concentration of L-FABP in the healthy serum measured by using the reference method was less than 3 ng/mL.

### (Measurement Conditions of LTIA)

(1) Analyzing device: Hitachi 7170 Automatic Analyzer (manufactured by Hitachi High-Technologies Corporation)
(2) Sample amount and reagent amounts: 3 µL of sample, 150 µL of the first reagent, 50 µL of the second reagent
(3) Reaction time (reaction temperature): 5 minutes (37 °C) for the first reagent, 5 minutes (37 °C) for the second reagent
(4) Photometric point and photometric object: absorbance changes between immediately after addition of the second reagent and 5 minutes after the addition

### [Example 1] Confirmation of Effects of Pretreatment Liquid of Reference Method and Compound of the Present Invention on LTIA

LTIA according to a conventional technique (Control Example A), LTIA using a pretreatment liquid of the reference method (Comparative Example 1), and LTIA using the compound of Formula (1) (benzamidine hydrochloride) (Example 1) were compared.

### 1. Operation

### (1) Example 1

L-FABP in the standard solution as a sample was measured by using the control first reagent containing the 500 mmol/L compound of Formula (1) (benzamidine hydrochloride) and the second reagent.

### (2) Control Example A

L-FABP in the standard solution as a sample was measured by using the control first reagent and the second reagent.

### (3) Comparative Example 1

The standard substance was diluted and adjusted to a desired concentration by using a solution acquired by mixing the pretreatment liquid of the reference method and a two-fold concentrated standard substance diluent at a ratio of 1 volume to 1 volume and was used as Sample for Comparative Example 1. L-FABP in Sample for Comparative Example 1 as a sample was measured by using the control first reagent and the second reagent.

### 2. Results

From the absorbance of the samples measured in the respective tests of Example 1, Control Example A, and Comparative Example 1, an absorbance of a 0 ng/mL L-FABP sample (blank absorbance) measured in each of the tests was subtracted to calculate the net absorbance. Fig. 1 shows calibration curves with the L-FABP concentration on the x-axis and the net absorbance on the y-axis.

When Sample for Comparative Example 1 containing the pretreatment liquid component of the reference method was measured (-■-), the net absorbance was lower than that of Control Example A (-◆-) at any L-FABP concentration and, moreover, no L-FABP concentration-dependent increase in absorbance was observed at all. As a result, a measurement interference effect of the component in the pretreatment liquid of the reference method on LTIA was confirmed. It is described in the package insert of the reference method that the pretreatment liquid contains a surfactant, and it was considered that the surfactant may be involved in the interference with measurement of LTIA.

On the other hand, when measured under the condition of Example 1 (-Δ-) using the control first reagent containing the compound of Formula (1) (benzamidine hydrochloride), the net absorbance exceeds that of Control Example A (-◆-) at any L-FABP concentration and a concentration-dependent increase in net absorbance was observed. As a result, the sensitizing effect of the compound of Formula (1) (benzamidine hydrochloride) on LTIA was confirmed.

### [Example 2] Confirmation of Effect of Compound of the Present Invention on LTIA in the Case of Measurement of Freeze-Thawed Urine

The effect on LTIA was confirmed by using a compound having a partial structure (NH₂-C=N-) common to a compound of Formula (1) (benzamidine hydrochloride) and a compound of Formula (2) (2-amino-2-thiazoline hydrochloride) but having no cyclic structure, as a compound of Reference Examples.

### 1. Operation

### (1) Example 2a, Example 2b

Twenty-one frozen-thawed urine specimens (L-FABP concentration: 6 ng/mL to 365 ng/mL) were employed as a sample and L-FABP in the sample was measured by using the control first reagent containing 500 mmol/L benzamidine hydrochloride (Example 2a) or the control first reagent containing 500 mmol/L 2-amino-2-thiazoline hydrochloride (Example 2b), and the second reagent.

### (2) Control Example B

The frozen-thawed urine described above was employed as a sample and L-FABP in the sample was measured by using the control first reagent and the second reagent.

### (3) Reference Example 2a

The frozen-thawed urine described above was employed as a sample and L-FABP in the sample was measured by using the control first reagent containing 500 mmol/L guanidine hydrochloride and the second reagent.

### (4) Reference Example 2b

The frozen-thawed urine described above was employed as a sample and L-FABP in the sample was measured by using the control first reagent containing 500 mmol/L guanidine sulfamate and the second reagent.

### (5) Reference Example 2c

The frozen-thawed urine described above was employed as a sample and L-FABP in the sample was measured by using the control first reagent containing 500 mmol/L aminoguanidine hydrochloride and the second reagent.

### 2. Results

The net absorbance of the samples measured in the respective tests of Examples 2a, 2b, Control Example B, and Reference Examples 2a to 2c was converted into L-FABP concentration by using the calibration curve created by using the standard solution as a sample. By plotting the L-FABP concentrations of the samples obtained by using the reference method (reference measurement values) on the x-axis and the L-FABP concentrations obtained from the respective tests (test measurement values) on the y-axis, the correlation of the test measurement values with the reference measurement values was studied by the least-squares method and shown in Table 1.

**[Table 1]**

| | R² | Reg.Eq. |
|---|---|---|
| Ex.2a | 0.968 | y=1.516x+1.44 |
| Ex.2b | 0.917 | y=0.972x-17.49 |
| Ct.Ex.B | 0.458 | y=0.128x-0.03 |
| Ref.Ex.2a | 0.837 | y=0,665x+4.59 |
| Ref.Ex.2b | 0.772 | y=0.582x-3.25 |
| Ref.Ex.2c | 0.787 | y=0.566x-1.10 |

| | | |
|---|---|---|
| Ex.: Example Ct.Ex.: Control Example Ref.Ex.: Reference Example Ref.Eq: Regression Equation | | |

R² between the measurement value and the reference measurement value in Control Example B was as low as 0.458 and it was confirmed that the condition of Control Example B is not applicable to the measurement in which urine as a specimen of clinical examination is used as a sample. On the other hand, the correlation between the measurement values in Example 2a and the reference measurement values was preferable. The correlation between the measurement values in Example 2b and the reference measurement values was also preferable. Therefore, it was confirmed that the compound of Formula (1) and the compound of Formula (2) can produce a sensitizing effect without impairing the correlation with the reference measurement value and also can make the immunoreactivity of urinal L-FABP and the standard substance comparable.

The correlation between the measurement values in Reference Examples 2a to 2c and the reference measurement values was lower than that of Example 2a and Example 2b although exceeding that of Control Example B. This indicated the possibility that, in addition to the partial structure (NH₂-C=N-) common to the compounds of Reference Examples, the cyclic structure possessed by the compounds of Formulae (1) and (2) is involved in the effect of the compound of Formula (1) and the compound of Formula (2).

### [Example 3] Confirmation of Preferred Concentration of Compound of the Present invention in LTIA, Part 1

A preferred concentration of the compound of Formula (1) (benzamidine hydrochloride: BA) was studied.

### 1. Operation

### (1) Example 3

Twelve frozen-thawed urine specimens (L-FABP concentration: 6 ng/mL to 130 ng/mL) were employed as a sample and L-FABP in the sample was measured by using the control first reagent containing the compound of Formula (1) (benzamidine hydrochloride: BAH) at the concentration shown in Table 2, and the second reagent.

### 2. Results

The net absorbance of the samples measured in Example 3 was converted into L-FABP concentration by using the calibration curve created by using the standard solution as a sample. By plotting the L-FABP concentrations of the samples obtained by using the reference method (reference measurement values) on the x-axis and the L-FABP concentrations obtained from the respective tests (test measurement values) on the y-axis, the correlation of the test measurement values with the reference measurement values was studied by the least-squares method and shown in Table 2.

**[Table 2]**

| Cpd. | Conc. (mmol/L) | R² | Reg.Eq. |
|---|---|---|---|
| Fml.(1) :BAH | 400 | 0.911 | y=1. 106x+0. 02 |
| | 500 | 0.855 | y=1.403x+4.95 |
| | 600 | 0.829 | y=1.602x+8.87 |

| | | | |
|---|---|---|---|
| Cpd.: Compound Fml.: Formula Cone.: Concentration Reg.Eq.: Regression Equation | | | |

When the compound of Formula (1) was used, favorable correlation was confirmed at any concentration. From the variation in the regression equation, it is considered that the sensitivity can be adjusted while maintaining the correlation by controlling the use concentration of the compound of Formula (1).

### [Example 4] Addition and Recovery Test of L-FABP in Serum

### 1. Operation

An addition and recovery test sample was prepared by diluting the L-FABP standard substance with the two-fold concentrated standard substance diluent and with the healthy serum such that the added L-FABP concentration were achieved as shown in Table 3. The final concentration of the healthy serum in the sample is 1/2 of the initial concentration.

L-FABP of the sample was measured by using the control first reagent containing 500 mmol/L benzamidine hydrochloride and the second reagent. A recovery rate was obtained from the acquired measurement value.

### 2. Results

The recovery rate at each L-FABP concentration is shown in Table 3.

**[Table 3]**

| Added L-FABP concentration | Recovery rate |
|---|---|
| 5ng/mL | 89.0% |
| 10ng/mL | 86.0% |
| 50ng/mL | 93.9% |
| 100ng/mL | 97.9% |
| 200ng/mL | 96.0% |

A favorable recovery rate was acquired in the tested concentration range of added L-FABP. It was confirmed that the method of the present invention is not limited to when the sample is urine and that a desired effect can be acquired even when serum is used as a sample. Since the same effect as in the case of using urine as a sample was acquired also in serum containing larger amounts of various coexisting proteins as compared to urine, it is considered that the effect of the compound of the present invention is selective to L-FABP as compared to the action on the coexisting proteins.

For Examples 5 and 6, measurement was performed by using the following materials under the following conditions.

### (Anti-L-FABP Antibody-Immobilized Latex Particle Suspension)

### (1) Preparation of Clone L Antibody-Immobilized Latex Particle Suspension

To 13 mL of a 20 mmol/L Tris buffer solution (pH 8.5) containing 0.36 mg/mL of anti-L-FABP antibody Clone L (manufactured by CMIC HOLDINGS Co., Ltd.), 13 mL of a 1 % latex particle (manufactured by SEKISUI CHEMICAL CO., LTD.) suspension having an average particle diameter of 0.27 µm was added and stirred at 4 °C for two hours. This was followed by addition of 13 mL of a 20 mmol/L Tris buffer solution (pH 8.5) containing 0.5 % BSA and stirring at 4 °C for one hour. Subsequently, dialysis with a 5 mmol/L MOPS buffer solution (pH 7.0) was performed to acquire a Clone L antibody-immobilized latex particle suspension.

### (2) Preparation of Clone 1 Antibody-Immobilized Latex Particle Suspension

To 8 mL of a 5 mmol/L Tris buffer solution (pH 7.5) containing 0.54 mg/mL of anti-L-FABP antibody Clone 1 (manufactured by CMIC HOLDINGS Co., Ltd.), 8 mL of a 1 % latex particle (manufactured by SEKISUI CHEMICAL CO., LTD.) suspension having an average particle diameter of 0.25 µm was added and stirred at 4 °C for two hours. This was followed by addition of 8 mL of a 5 mmol/L Tris buffer solution (pH 7.5) containing 0.5 % BSA and stirring at 4 °C for one hour. Subsequently, dialysis with a 5 mmol/L MOPS buffer solution (pH 7.0) was performed to acquire a Clone 1 antibody-immobilized latex particle suspension.

### (L-FABP Standard Substance)

The L-FABP standard substance was acquired by gene recombination as described in Patent Document 1.

### (L-FABP Reference Measurement Method: Reference Method)

A pharmaceutical product for in-vitro diagnosis by ELISA (Renapro (registered trademark) L-FABP test TMB) was used for a reference method.

### (Control First Reagent)

300 mmol/L KCl
0.2% BPF (manufactured by TOYOBO, Catalog No. BPF-301)
0.32 to 0.68% Lipidure-BL403SE

### (Second Reagent)

5 mmol/L MOPS buffer solution (pH 7.0)
3.75 Abs/mL Clone L antibody-immobilized latex particle suspension^{(*)}
1.25 Abs/mL Clone 1 antibody-immobilized latex particle suspension^{(*)}
^{(*)} Abs denotes the absorbance at 280 nm.

### (Standard Substance Diluent)

Phosphate buffer solution (pH 7.0)
0.1% BPF (manufactured by TOYOBO, Catalog No. BPF-301)

### (Standard Solution)

The L-FABP standard substance was adjusted to a desired concentration by using the standard substance diluent and was used as a standard solution.

### (Freeze-Thawed Urine)

Partial urine frozen and stored at -30 °C after collection was thawed only once and used for measurement.

### (Measurement Conditions of LTIA)

(1) Analyzing device: Hitachi 7170 Automatic Analyzer (manufactured by Hitachi High-Technologies Corporation)
(2) Sample amount and reagent amounts: 3 µL of sample, 150 µL of the first reagent, 50 µL of the second reagent
(3) Reaction time (reaction temperature): 5 minutes (37 °C) for the first reagent, 5 minutes (37 °C) for the second reagent
(4) Photometric point and photometric object: absorbance changes between immediately after addition of the second reagent and 5 minutes after the addition
(5) Measurement wavelength 570 nm/800 nm

### [Example 5] Confirmation of Preferred Concentration of Compound of the Present invention in LTIA, Part 2

The effect on LTIA was confirmed by using the compound of Formula (1) (benzamidine hydrochloride) and the compound of Formula (2) (2-amino-2-thiazoline hydrochloride and creatinine).

### 1. Operation

### (1) Examples 5a, 5b, and 5c

Thirty-four frozen-thawed urine specimens (L-FABP concentration: 0.6 ng/mL to 123.0 ng/mL) were employed as a sample and L-FABP in the sample was measured by using the control first reagent containing benzamidine hydrochloride (structural formula: 5a in Table 5, Example 5a), 2-amino-2-thiazoline hydrochloride (structural formula: 5b in Table 5, Example 5b), or creatinine (structural formula: 5c in Table 5, Example 5c) at concentration shown in Table 4, and the second reagent.

### 2. Results

The net absorbance of the samples measured in the respective tests of Examples 5a, 5b, 5c was converted into L-FABP concentration by using the calibration curve created by using the standard solution as a sample. By plotting the L-FABP concentrations of the samples obtained by using the reference method (reference measurement values) on the x-axis and the L-FABP concentrations obtained from the respective tests (test measurement values) on the y-axis, the correlation of the test measurement values with the reference measurement values was studied by the least-squares method and shown in Table 4.

**[Table 4]**

| | **mmol/L** | **R²** |
|---|---|---|
| Benzamidine hydrochloride (**5a**) | **300** | **0.9763** |
| | **400** | **0.9727** |
| | **450** | **0.9697** |
| | **500** | **0.9715** |
| | **600** | **0.9711** |
| 2-amino-2-thiazoline hydrochloride (**5b**) | **300** | **0.9050** |
| | **400** | **0.9394** |
| | **450** | **0.9474** |
| | **500** | **0.9609** |
| | **600** | **0.9810** |
| Creatinine (**5c**) | **300** | **0.8551** |
| | **400** | **0.8613** |
| | **450** | **0.8636** |
| | **500** | **0.8536** |
| | **600** | **0.8883** |

**[Table 5]**

| | Structural formula |
|---|---|
| 5a | 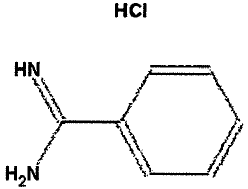 |
| 5b | 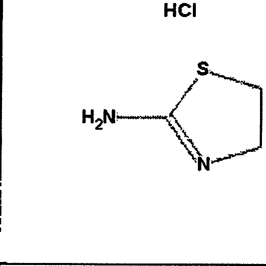 |
| 5c | 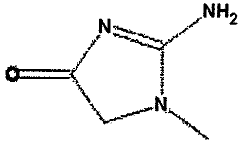 |

The correlation between the measurement values in Examples 5a, 5b, 5c and the reference measurement values was preferable at any concentration. Additionally, it was confirmed that the immunoreactivity of urinal L-FABP and the standard substance can be made comparable.

### [Example 6] Confirmation of Effect of Compound of the Present Invention on Sample Storage Condition

It is described in the package insert of the reference method, Renapro (registered trademark) L-FABP test TMB that when a sample is stored after urine is collected as the sample, the sample should be refrigerated or frozen (-20 to -80 °C).

In a clinical setting, measurement cannot be performed immediately after a sample is collected in some cases. Inappropriate storage of a sample may lead to unexpected variation in measurement value. A sample (urine) stored at room temperature for 24 hours was measured by using the reagent of the present invention.

### 1. Operation

### (1) Example 6, Comparative Example 2

Twenty-three frozen-thawed urine specimens (L-FABP concentration: 0.3 ng/mL to 111.9 ng/mL) were employed as a sample and L-FABP in the sample was measured by using the control first reagent containing the compound of Formula (1) (benzamidine hydrochloride: BAH) at the concentration shown in Table 6 (Example 6, Comparative Example 2) and the second reagent. For Reference Example, L-FABP was also measured by using the same reagents for the samples stored under the conditions described in the package insert of the reference method (refrigerated storage for 24 hours).

### 2. Results

The net absorbance of the samples measured in the respective tests of Example 6, Comparative Example 2, and Reference Example was converted into L-FABP concentration by using the calibration curve created by using the standard solution as a sample. By plotting the L-FABP concentrations of the samples obtained by using the samples stored for zero hour (zero-hour measurement values) on the x-axis and the L-FABP concentrations obtained from the respective tests (test measurement values) on the y-axis, the correlation of the test measurement values with the zero-hour measurement values was studied by the least-squares method and shown in Table 6.

**[Table 6]**

| | Storage state of sample | Cpd. of Fml (1) | R² | Regression equation |
|---|---|---|---|---|
| Ex.6 | RT 24hr | 500mmol/L BAH | 0.9723 | y=1.1236x -0.2944 |
| Cp.Ex.2 | | 250 mmol/L BAH | 0.9631 | y=1.8641 x-1.1744 |
| Ref.Ex. | Rfg. 24hr | 500 mmol/L BAH | 0.9977 | y=1.0486 x-0.3752 |
| | | 250 mmol/L BAH | 0.9979 | y=1.0987x +0.2619 |

| | | | | |
|---|---|---|---|---|
| Ex.6: Example 6 Cp.Ex.2: Comparative Example 2 Ref.Ex.: Reference Example RT 24hr: At room temperature for 24 hours Rfg. 24hr: Refrigerated for 24 hours Cpd. of Fml (1): Compound of Formula (1) Regression equation | | | | |

When the sample stored at room temperature for 24 hours was measured with the reagent to which 250 mmol/L benzamidine hydrochloride was added, the correlation with the zero-hour storage was favorable; however, the slope of the regression equation was large. This indicates that the measurement value of the sample stored at room temperature for 24 hours is higher than the measurement value of the zero-hour storage. On the other hand, when the sample was measured with the reagent to which 500 mmol/L benzamidine hydrochloride was added, the correlation with the zero-hour storage was favorable, and the variation in the slope of the regression equation was within 15 %. Even in the case of the sample stored at room temperature for 24 hours, which is the condition not described in the package insert of the reference method, L-FABP in the sample can be measured by using the reagent of the present invention, as is the case with a fresh sample or a sample in a favorable storage condition.

It was also indicated that the compound of the present invention can be used as a storage solution for a sample.

### INDUSTRIAL APPLICABILITY

According to the present invention, by bringing the compound having a partial structure of NH₂-C=N- and a cyclic structure in a molecule into contact with L-FABP in a sample, L-FABP in the sample can quickly and easily be detected with an immunological measurement method.

## Claims

1. A method of detecting L-FABP (liver-type fatty acid binding protein) in a sample with an anti-L-FABP antibody by a particle agglutination measurement method , comprising the step of: bringing an anti-L-FABP antibody and a compound having a partial structure of NH₂-C=N- and a cyclic structure in a molecule into contact with a sample suspected of containing L-FABP, wherein
the step of bringing the anti-L-FABP antibody and the compound having a partial structure of NH₂-C=N- and a cyclic structure in a molecule into contact with the sample suspected of containing L-FABP is performed in the order in which
a step of bringing the compound into contact with the sample suspected of containing L-FABP is followed by a step of bringing the compound into contact with the anti-L-FABP antibody;
wherein the compound having a partial structure of NH₂-C=N- and a cyclic structure in a molecule is one or two or more selected from a compound represented by Formula (1) or salt or ester thereof and a compound represented by Formula (2) or salt thereof:
the compound of Formula (1) 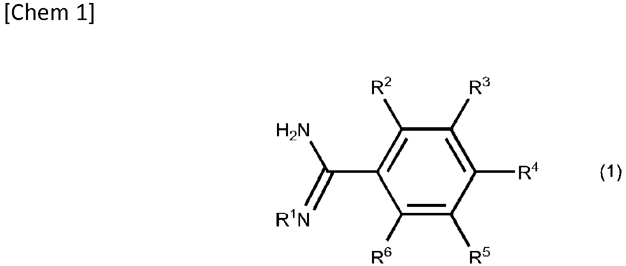 [in Formula (1), R¹ is a hydrogen atom, a hydroxyl group, or an alkyl group having the carbon number of 1, 2, or 3 that may be branched, and R² to R⁶ each independently represent a hydrogen atom, a halogen atom, an alkyl group having the carbon number of 1, 2, or 3 that may be branched, a hydroxyl group, a carboxy group, an amino group, or -SR⁷ (R⁷ represents a hydrogen atom, a hydroxyl group, or an alkyl group having the carbon number of 1, 2, or 3 that may be branched and, when a plurality of R⁷s are present, R⁷s may be the same groups as or different groups from each other)] or salt or ester thereof; and
the compound of Formula (2) 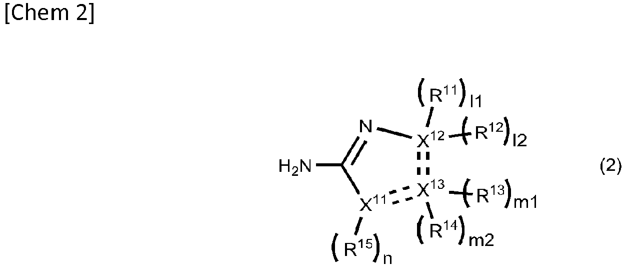
[in Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together, R¹⁵ represents a hydrogen atom, a halogen atom, or an alkyl group having the carbon number of 1, 2, or 3 that may be branched,
X¹¹ is a nitrogen atom or a sulfur atom,
X¹² and X¹³ are each independently a carbon atom or a nitrogen atom, and
l1, l2, m1, m2, and n are each independently 0 or 1,
a double broken line between X¹¹ and X¹³ and a double broken line between X¹² and X¹³ are each independently a single bond or a double bond, wherein
the values of l1, l2, m1, m2, and n as well as the bonds of the double broken line between X¹¹ and X¹³ and the double broken line between X¹² and X¹³ indicate values and bonds appropriately determined depending on the valences of X¹¹ to X¹³], or salt thereof.

2. The method according to claim 1, wherein the compound represented by Formula (2) or salt thereof is one or two or more selected from the following compound or salt thereof:
the compound of Formula (2) 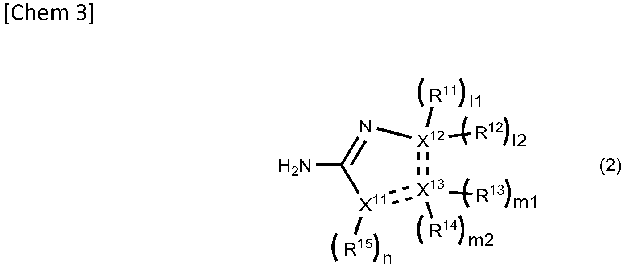
[In Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together, R¹⁵ represents a hydrogen atom, a halogen atom, or an alkyl group having the carbon number of 1, 2, or 3 that may be branched], wherein
in a combination of X¹¹ to X¹³, l1+l12, m1+m2, n (l1, l2, m1, m2, and n each independently represent 0 or 1), and double broken lines,
(a) X¹¹ is a sulfur atom, X¹² and X¹³ are carbon atoms, l1+l2 is 2, m1+m2 is 2, n is 0, and the double broken lines between X¹¹ and X¹³ and between X¹² and X¹³ are single bonds,
(b) X¹¹ is a sulfur atom, X¹² and X¹³ are carbon atoms, l1+l2 is 1, m1+m2 is 1, n is 0, the double broken line between X¹¹ and X¹³ is a single bond, and the double broken line between X¹² and X¹³ is a double bond,
(c) X¹¹ is a nitrogen atom, X¹² and X¹³ are carbon atoms, l1+l2 is 2, m1+m2 is 2, n is 1, and the double broken lines between X¹¹ and X¹³ and between X¹² and X¹³ are single bonds,
(d) X¹¹ is a nitrogen atom, X¹² and X¹³ are carbon atoms, l1+l2 is 1, m1+m2 is 1, n is 1, the double broken line between X¹¹ and X¹³ is a single bond, and the double broken line between X¹² and X¹³ is a double bond,
(e) X¹¹ and X¹² are nitrogen atoms, X¹³ is a carbon atom, l1+l2 is 1, m1+m2 is 1, n is 0, the double broken line between X¹¹ and X¹³ is a double bond, and the double broken line between X¹² and X¹³ is a single bond, or
(f) X¹¹, X¹², and X¹³ are nitrogen atoms, l1+l2 is 0, m1+m2 is 0, n is 1, the double broken line between X¹¹ and X¹³ is a single bond, and the double broken line between X¹² and X¹³ is a double bond, or salt thereof.

3. The method according to any one of claims 1 to 2, wherein at the step of bringing the anti-L-FABP antibody and the compound having a partial structure of NH₂-C=N- and a cyclic structure in a molecule into contact with a sample suspected of containing L-FABP, the concentration of the compound is 300 mmol/L to 500 mmol/L.

4. The method according to any one of claims 1 to 3, wherein the anti-L-FABP antibody is immobilized on an insoluble carrier, and the insoluble carrier is a latex particle, or a metal colloid particle.

5. The method according to any one of claims 1 to 4, wherein the anti-L-FABP antibody is two or more monoclonal antibodies having recognition sites different from each other.

6. The method according to claim 5, wherein the two or more monoclonal antibodies having recognition sites different from each other are respectively immobilized on latex particles, and wherein L-FABP is detected by a latex turbidimetric immunoassay.

7. The method according to any one of claims 1 to 6, wherein the sample is urine, whole blood, serum, or plasma.

8. A method of improving measurement sensitivity in a method of detecting L-FABP in a sample with an anti-L-FABP antibody by a particle agglutination measurement method, comprising the step of: bringing an anti-L-FABP antibody and a compound having a partial structure of NH₂-C=N- and a cyclic structure in a molecule into contact with a sample suspected of containing L-FABP, wherein
the step of bringing the anti-L-FABP antibody and the compound having a partial structure of NH₂-C=N- and a cyclic structure in a molecule into contact with the sample suspected of containing L-FABP is performed in the order in which
a step of bringing the compound into contact with the sample suspected of containing L-FABP is followed by a step of bringing the compound into contact with the anti-L-FABP antibody;
wherein the compound having a partial structure of NH₂-C=N- and a cyclic structure in a molecule is one or two or more selected from a compound represented by Formula (1) or salt or ester thereof and a compound represented by Formula (2) or salt thereof:
the compound of Formula (1) 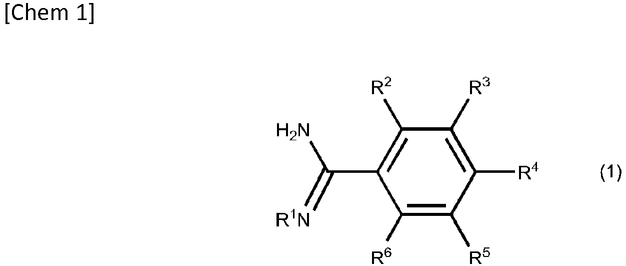 [in Formula (1), R¹ is a hydrogen atom, a hydroxyl group, or an alkyl group having the carbon number of 1, 2, or 3 that may be branched, and R² to R⁶ each independently represent a hydrogen atom, a halogen atom, an alkyl group having the carbon number of 1, 2, or 3 that may be branched, a hydroxyl group, a carboxy group, an amino group, or -SR⁷ (R⁷ represents a hydrogen atom, a hydroxyl group, or an alkyl group having the carbon number of 1, 2, or 3 that may be branched and, when a plurality of R⁷s are present, R⁷s may be the same groups as or different groups from each other)] or salt or ester thereof; and
the compound of Formula (2) 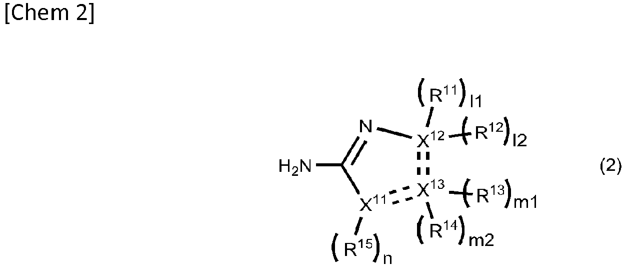
[in Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together, R¹⁵ represents a hydrogen atom, a halogen atom, or an alkyl group having the carbon number of 1, 2, or 3 that may be branched,
X¹¹ is a nitrogen atom or a sulfur atom,
X¹² and X¹³ are each independently a carbon atom or a nitrogen atom, and
l1, l2, m1, m2, and n are each independently 0 or 1,
a double broken line between X¹¹ and X¹³ and a double broken line between X¹² and X¹³ are each independently a single bond or a double bond, wherein
the values of l1, l2, m1, m2, and n as well as the bonds of the double broken line between X¹¹ and X¹³ and the double broken line between X¹² and X¹³ indicate values and bonds appropriately determined depending on the valences of X¹¹ to X¹³], or salt thereof.

## Patentansprüche

1. Verfahren zum Detektieren von L-FABP (Leber-Typ-Fettsäure-Bindungsprotein (liver-type fatty acid binding protein)) in einer Probe mit einem Anti-L-FABP-Antikörper durch ein Partikel-Agglutinationsmessverfahren, umfassend den folgenden Schritt: Inkontaktbringen eines Anti-L-FABP-Antikörpers und einer Verbindung mit einer partiellen Struktur von NH₂-C=N- und einer zyklischen Struktur in einem Molekül mit einer Probe, von der vermutet wird, dass sie L-FABP enthält, wobei
der Schritt des Inkontaktbringens des Anti-L-FABP-Antikörpers und der Verbindung mit einer partiellen Struktur von NH₂-C=N- und einer zyklischen Struktur in einem Molekül mit der Probe, von der vermutet wird, dass sie L-FABP enthält, in der Reihenfolge durchgeführt wird, in der
auf einen Schritt des Inkontaktbringens der Verbindung mit der Probe, von der vermutet wird, dass sie L-FABP enthält, ein Schritt des Inkontaktbringens der Verbindung mit dem Anti-L-FABP-Antikörper folgt;
wobei die Verbindung mit einer partiellen Struktur von NH₂-C=N- und einer zyklischen Struktur in einem Molekül eine oder zwei oder mehr ist, ausgewählt aus einer Verbindung, repräsentiert durch Formel (1), oder einem Salz oder Ester davon, und einer Verbindung, repräsentiert durch Formel (2), oder einem Salz davon:
die Verbindung der Formel (1) 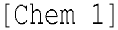 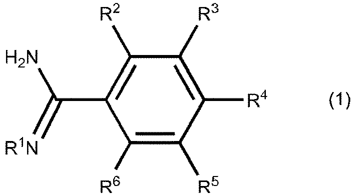 [in Formel (1) ist R¹ ein Wasserstoffatom, eine Hydroxylgruppe oder eine Alkylgruppe mit einer Kohlenstoffanzahl von 1, 2 oder 3, die verzweigt sein kann, und R² bis R⁶ repräsentieren jeweils unabhängig ein Wasserstoffatom, ein Halogenatom, eine Alkylgruppe mit einer Kohlenstoffanzahl von 1, 2 oder 3, die verzweigt sein kann, eine Hydroxylgruppe, eine Carboxygruppe, eine Aminogruppe oder -SR⁷ (R⁷ repräsentiert ein Wasserstoffatom, eine Hydroxylgruppe oder eine Alkylgruppe mit einer Kohlenstoffanzahl von 1, 2 oder 3, die verzweigt sein kann, und wenn eine Mehrzahl von R⁷s vorliegt, können R⁷s die gleichen Gruppen oder sich voneinander unterscheidende Gruppen sein)] oder ein Salz oder ein Ester davon; und
die Verbindung der Formel (2) 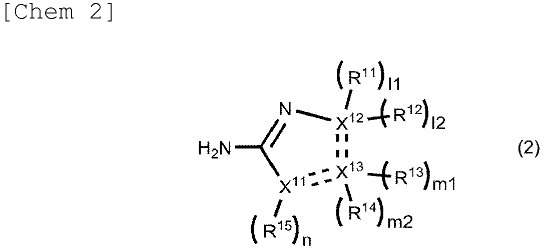
[in Formel (2) repräsentieren R¹¹ bis R¹⁴ jeweils unabhängig ein Wasserstoffatom, ein Halogenatom, eine Alkylgruppe mit einer Kohlenstoffanzahl von 1, 2 oder 3, die verzweigt sein kann, eine Aminogruppe, eine Phenylgruppe, die mit einem Halogenatom substituiert sein kann, oder -SR¹⁶ (R¹⁶ repräsentiert ein Wasserstoffatom, eine Hydroxylgruppe oder eine Alkylgruppe mit einer Kohlenstoffanzahl von 1, 2 oder 3, die verzweigt sein kann, und wenn eine Mehrzahl von R¹⁶s vorliegt, können R¹⁶s die gleichen Gruppen oder sich voneinander unterscheidende Gruppen sein), wobei R¹¹ und R¹², die in dem gleichen Molekül vorliegen, zusammen eine Carbonylgruppe bilden können, und R¹³ und R¹⁴, die in dem gleichen Molekül vorliegen, zusammen eine Carbonylgruppe bilden können, R¹⁵ repräsentiert ein Wasserstoffatom, ein Halogenatom oder eine Alkylgruppe mit einer Kohlenstoffanzahl von 1, 2 oder 3, die verzweigt sein kann,
X¹¹ ist ein Stickstoffatom oder ein Schwefelatom,
X¹² und X¹³ sind jeweils unabhängig ein Kohlenstoffatom oder ein Stickstoffatom, und
l1, l2, m1, m2 und n sind jeweils unabhängig 0 oder 1,
eine doppelte, unterbrochene Linie zwischen X¹¹ und X¹³ und eine doppelte, unterbrochene Linie zwischen X¹² und X¹³ sind jeweils unabhängig eine Einfachbindung oder eine Doppelbindung, wobei
die Werte l1, l2, m1, m2 und n sowie die Bindungen der doppelten, unterbrochenen Linie zwischen X¹¹ und X¹³ und der doppelten, unterbrochenen Linie zwischen X¹² und X¹³ weisen auf Werte und Bindungen hin, die in Abhängigkeit von den Valenzen von X¹¹ bis X¹³ in angemessener Weise bestimmt wurden], oder ein Salz davon.

2. Verfahren gemäß Anspruch 1, wobei die durch Formel (2) repräsentierte Bindung oder das Salz davon eine oder zwei oder mehr ist, ausgewählt aus der folgenden Verbindung oder dem Salz davon:
die Verbindung von Formel (2) 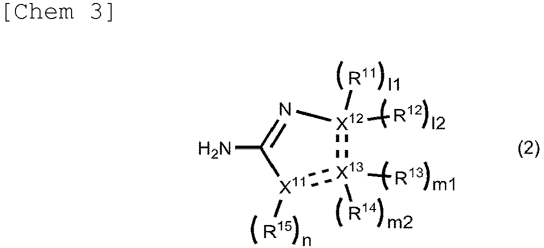
[in Formel (2) repräsentieren R¹¹ bis R¹⁴ jeweils unabhängig ein Wasserstoffatom, ein Halogenatom, eine Alkylgruppe mit einer Kohlenstoffanzahl von 1, 2 oder 3, die verzweigt sein kann, eine Aminogruppe, eine Phenylgruppe, die mit einem Halogenatom substituiert sein kann, oder -SR¹⁶ (R¹⁶ repräsentiert ein Wasserstoffatom, eine Hydroxylgruppe oder eine Alkylgruppe mit einer Kohlenstoffanzahl von 1, 2 oder 3, die verzweigt sein kann, und wenn eine Mehrzahl von R¹⁶s vorliegt, können R¹⁶s die gleichen Gruppen oder sich voneinander unterscheidende Gruppen sein), wobei R¹¹ und R¹², die in dem gleichen Molekül vorliegen, zusammen eine Carbonylgruppe bilden können, und R¹³ und R¹⁴, die in dem gleichen Molekül vorliegen, zusammen eine Carbonylgruppe bilden können, R¹⁵ repräsentiert ein Wasserstoffatom, ein Halogenatom oder eine Alkylgruppe mit einer Kohlenstoffanzahl von 1, 2 oder 3, die verzweigt sein kann], wobei
in einer Kombination von X¹¹ bis X¹³, l1+l2, m1+m2, n (l1, l2, m1, m2 und n repräsentieren jeweils unabhängig 0 oder 1) und doppelten, unterbrochenen Linien
(a) X¹¹ ein Schwefelatom ist, X¹² und X¹³ Kohlenstoffatome sind, l1+l2 2 ist, m1+m2 2 ist, n 0 ist und die doppelten, unterbrochenen Linien zwischen X¹¹ und X¹³ und zwischen X¹² und X¹³ Einfachbindungen sind,
(b) X¹¹ ein Schwefelatom ist, X¹² und X¹³ Kohlenstoffatome sind, l1+l2 1 ist, m1+m2 1 ist, n 0 ist, die doppelte, unterbrochene Linie zwischen X¹¹ und X¹³ eine Einfachbindung ist und die doppelte, unterbrochene Linie zwischen X¹² und X¹³ eine Doppelbindung ist,
(c) X¹¹ ein Stickstoffatom ist, X¹² und X¹³ Kohlenstoffatome sind, l1+l2 2 ist, m1+m2 2 ist, n 1 ist und die doppelten, unterbrochenen Linien zwischen X¹¹ und X¹³ und zwischen X¹² und X¹³ Einfachbindungen sind,
(d) X¹¹ ein Stickstoffatom ist, X¹² und X¹³ Kohlenstoffatome sind, 11+12 1 ist, m1+m2 1 ist, n 1 ist, die doppelte, unterbrochene Linie zwischen X¹¹ und X¹³ eine Einfachbindung ist und die doppelte, unterbrochene Linie zwischen X¹² und X¹³ eine Doppelbindung ist,
(e) X¹¹ und X¹² Stickstoffatome sind, X¹³ ein Kohlenstoffatom ist, l1+l2 1 ist, m1+m2 1 ist, n 0 ist, die doppelte, unterbrochene Linie zwischen X¹¹ und X¹³ eine Doppelbindung ist und die doppelte, unterbrochene Linie zwischen X¹² und X¹³ eine Einfachbindung ist, oder
(f) X¹¹, X¹² und X¹³ Stickstoffatome sind, l1+l2 0 ist, m1+m2 0 ist, n 1 ist, die doppelte, unterbrochene Linie zwischen X¹¹ und X¹³ eine Einfachbindung ist und die doppelte, unterbrochene Linie zwischen X¹² und X¹³ eine Doppelbindung ist, oder ein Salz davon.

3. Verfahren gemäß irgendeinem der Ansprüche 1 bis 2, wobei in dem Schritt des Inkontaktbringens des Anti-L-FABP-Antikörpers und der Verbindung, die eine partielle Struktur von NH₂-C=N- und eine zyklische Struktur in einem Molekül hat, mit einer Probe, von der vermutet wird, dass sie L-FABP enthält, die Konzentration der Verbindung 300 mmol/l bis 500 mmol/l ist.

4. Verfahren gemäß irgendeinem der Ansprüche 1 bis 3, wobei der Anti-L-FABP-Antikörper auf einem unlöslichen Träger immobilisiert ist und der unlösliche Träger ein Latexpartikel oder ein Metallkolloidpartikel ist.

5. Verfahren gemäß irgendeinem der Ansprüche 1 bis 4, wobei der Anti-L-FABP-Antikörper zwei oder mehr monoklonale Antikörper mit sich voneinander unterscheidenden Erkennungsstellen ist.

6. Verfahren gemäß Anspruch 5, wobei die zwei oder mehr monoklonalen Antikörper mit sich voneinander unterscheidenden Erkennungsstellen jeweils auf Latexpartikeln immobilisiert sind, und wobei L-FABP durch einen turbidimetrischen Latex-Immunassay detektiert wird.

7. Verfahren gemäß irgendeinem der Ansprüche 1 bis 6, wobei die Probe Urin, Vollblut, Serum oder Plasma ist.

8. Verfahren zum Verbessern der Messempfindlichkeit in einem Verfahren zum Detektieren von L-FABP in einer Probe mit einem Anti-L-FABP-Antikörper durch ein Partikel-Agglutinationsmessverfahren, umfassend den folgenden Schritt: Inkontaktbringen eines Anti-L-FABP-Antikörpers und einer Verbindung mit einer partiellen Struktur von NH₂-C=N- und einer zyklischen Struktur in einem Molekül mit einer Probe, von der vermutet wird, dass sie L-FABP enthält, wobei
der Schritt des Inkontaktbringens des Anti-L-FABP-Antikörpers und der Verbindung, die eine partielle Struktur von NH₂-C=N- und eine zyklische Struktur in einem Molekül hat, mit der Probe, von der vermutet wird, dass sie L-FABP enthält, in der Reihenfolge durchgeführt wird, in der
auf einen Schritt des Inkontaktbringens der Verbindung mit der Probe, von der vermutet wird, dass sie L-FABP enthält, ein Schritt des Inkontaktbringens der Verbindung mit dem Anti-L-FABP-Antikörper folgt;
wobei die Verbindung, die eine partielle Struktur von NH₂-C=N- und eine zyklische Struktur in einem Molekül hat, eine oder zwei oder mehr ist, ausgewählt aus einer Verbindung, repräsentiert durch Formel (1), oder einem Salz oder einem Ester davon, und einer Verbindung, repräsentiert durch Formel (2), oder einem Salz davon:
die Verbindung der Formel (1): 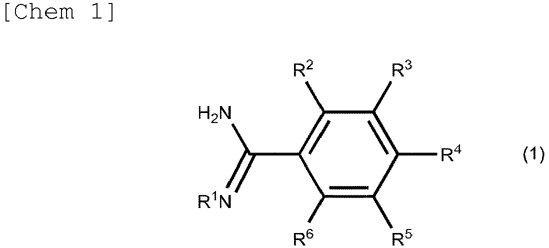 [in Formel (1) ist R¹ ein Wasserstoffatom, eine Hydroxylgruppe oder eine Alkylgruppe mit einer Kohlenstoffanzahl von 1, 2 oder 3, die verzweigt sein kann, und R² bis R⁶ repräsentieren jeweils unabhängig ein Wasserstoffatom, ein Halogenatom, eine Alkylgruppe mit einer Kohlenstoffanzahl von 1, 2 oder 3, die verzweigt sein kann, eine Hydroxylgruppe, eine Carboxygruppe, eine Aminogruppe oder -SR⁷ (R⁷ repräsentiert ein Wasserstoffatom, eine Hydroxylgruppe oder eine Alkylgruppe mit einer Kohlenstoffanzahl von 1, 2 oder 3, die verzweigt sein kann, und wenn eine Mehrzahl von R⁷s vorliegt, können R⁷s die gleichen Gruppen oder sich voneinander unterscheidende Gruppen sein)] oder ein Salz oder Ester davon; und
die Verbindung der Formel (2) 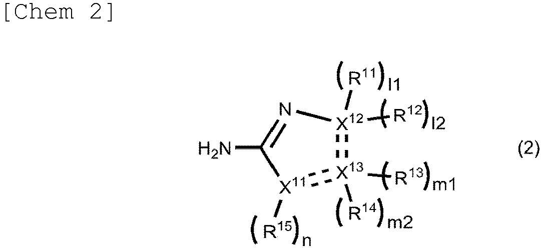
[in Formel (2) repräsentieren R¹¹ bis R¹⁴ jeweils unabhängig ein Wasserstoffatom, ein Halogenatom, eine Alkylgruppe mit einer Kohlenstoffanzahl von 1, 2 oder 3, die verzweigt sein kann, eine Aminogruppe, eine Phenylgruppe, die mit einem Halogenatom substituiert sein kann, oder -SR¹⁶ (R¹⁶ repräsentiert ein Wasserstoffatom, eine Hydroxylgruppe oder eine Alkylgruppe mit einer Kohlenstoffanzahl von 1, 2 oder 3, die verzweigt sein kann, und wenn eine Mehrzahl von R¹⁶s vorliegt, können R¹⁶s die gleichen Gruppen oder sich voneinander unterscheidende Gruppen sein), wobei R¹¹ und R¹², die in dem gleichen Molekül vorliegen, zusammen eine Carbonylgruppe bilden können, und R¹³ und R¹⁴, die in dem gleichen Molekül vorliegen, zusammen eine Carbonylgruppe bilden können, R¹⁵ repräsentiert ein Wasserstoffatom, ein Halogenatom oder eine Alkylgruppe mit einer Kohlenstoffanzahl von 1, 2 oder 3, die verzweigt sein kann,
X¹¹ ist ein Stickstoffatom oder ein Schwefelatom,
X¹² und X¹³ sind jeweils unabhängig ein Kohlenstoffatom oder ein Stickstoffatom, und
11, 12, m1, m2 und n sind jeweils unabhängig 0 oder 1,
eine doppelte, unterbrochene Linie zwischen X¹¹ und X¹³ und eine doppelte, unterbrochene Linie zwischen X¹² und X¹³ sind jeweils unabhängig eine Einfachbindung oder eine Doppelbindung, wobei
die Werte von 11, 12, m1, m2 und n sowie die Bindungen der doppelten, unterbrochenen Linie zwischen X¹¹ und X¹³ und der doppelten, unterbrochenen Linie zwischen X¹² und X¹³ auf Werte und Bindungen hindeuten, die in Abhängigkeit von den Valenzen von X¹¹ bis X¹³ in angemessener Weise bestimmt wurden], oder ein Salz davon.

## Revendications

1. Procédé de détection de L-FABP (protéine de fixation d'acide gras de type hépatique) dans un échantillon avec un anticorps anti-L-FABP par un procédé de mesure d'agglutination de particules, comprenant l'étape consistant à : amener un anticorps anti-L-FABP et un composé ayant une structure partielle de NH₂-C=N- et une structure cyclique dans une molécule en contact avec un échantillon suspecté de contenir L-FABP, dans lequel
l'étape consistant à amener l'anticorps anti-L-FABP et le composé ayant une structure partielle de NH₂-C=N- et une structure cyclique dans une molécule en contact avec l'échantillon suspecté de contenir L-FABP est réalisée dans l'ordre dans lequel
une étape consistant à amener le composé en contact avec l'échantillon suspecté de contenir L-FABP est suivie par une étape consistant à amener le composé en contact avec l'anticorps anti-L-FABP ;
dans lequel le composé ayant une structure partielle de NH₂-C=N- et une structure cyclique dans une molécule est un ou deux ou plus de deux éléments choisis parmi un composé représenté par la formule (1) ou un sel ou ester de celui-ci et un composé représenté par la formule (2) ou un sel de celui-ci :
le composé de formule (1) 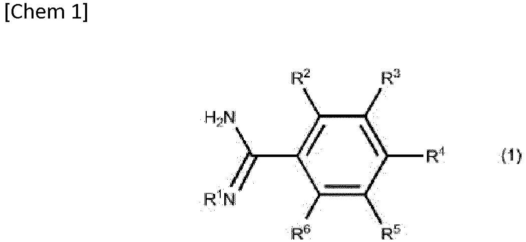 [dans la formule (1), R¹ est un atome d'hydrogène, un groupe hydroxyle, ou un groupe alkyle dont le nombre d'atomes de carbone est de 1, 2, ou 3 qui peut être ramifié, et R² à R⁶ représentent chacun indépendamment un atome d'hydrogène, un atome d'halogène, un groupe alkyle dont le nombre d'atomes de carbone est de 1, 2, ou 3 qui peut être ramifié, un groupe hydroxyle, un groupe carboxy, un groupe amino, ou -SR⁷ (R⁷ représente un atome d'hydrogène, un groupe hydroxyle, ou un groupe alkyle dont le nombre d'atomes de carbone est de 1, 2, ou 3 qui peut être ramifié et, lorsqu'une pluralité de R⁷ sont présents, les R⁷ peuvent être les mêmes groupes ou des groupes différents les uns des autres)] ou un sel ou ester de ceux-ci ; et
le composé de formule (2) 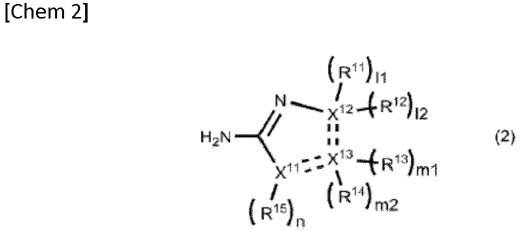
[dans la formule (2), R¹¹ à R¹⁴ représentent chacun indépendamment un atome d'hydrogène, un atome d'halogène, un groupe alkyle dont le nombre d'atomes de carbone est de 1, 2, ou 3 qui peut être ramifié, un groupe amino, un groupe phényle qui peut être substitué par un atome d'halogène, ou -SR¹⁶ (R¹⁶ représente un atome d'hydrogène, un groupe hydroxyle, ou un groupe alkyle dont le nombre d'atomes de carbone est de 1, 2, ou 3 qui peut être ramifié et, lorsqu'une pluralité de R¹⁶ sont présents, les R¹⁶ peuvent être des groupes identiques ou des groupes différents les uns des autres), dans lequel R¹¹ et R¹² présents dans la même molécule peuvent former un groupe carbonyle conjointement et R¹³ et R¹⁴ présents dans la même molécule peuvent former un groupe carbonyle conjointement, R¹⁵ représente un atome d'hydrogène, un atome d'halogène, ou un groupe alkyle dont le nombre d'atomes de carbone est de 1, 2, ou 3 qui peut être ramifié,
X¹¹ est un atome d'azote ou un atome de soufre,
X¹² et X¹³ sont chacun indépendamment un atome de carbone ou un atome d'azote, et
l1, l2, m1, m2, et n valent chacun indépendamment 0 ou 1,
un tiret double entre X¹¹ et X¹³ et un tiret double entre X¹² et X¹³ sont chacun indépendamment une simple liaison ou une double liaison, dans lequel
les valeurs de l1, l2, m1, m2, et n ainsi que les liaisons du tiret double entre X¹¹ et X¹³ et du tiret double entre X¹² et X¹³ indiquent des valeurs et des liaisons déterminées adéquatement selon les valences de X¹¹ à X¹³], ou sel de celui-ci.

2. Procédé selon la revendication 1, dans lequel le composé représenté par la formule (2) ou le sel de celui-ci est un ou deux ou plus de deux éléments choisis parmi le composé suivant ou un sel de celui-ci :
le composé de formule (2) 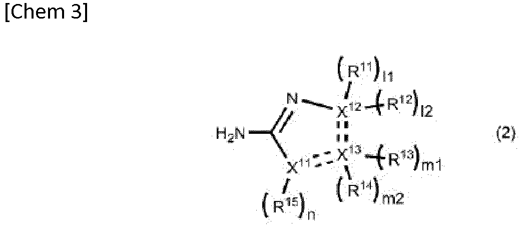
[dans la formule (2), R¹¹ à R¹⁴ représentent chacun indépendamment un atome d'hydrogène, un atome d'halogène, un groupe alkyle dont le nombre d'atomes de carbone est de 1, 2, ou 3 qui peut être ramifié, un groupe amino, un groupe phényle qui peut être substitué par un atome d'halogène, ou -SR¹⁶ (R¹⁶ représente un atome d'hydrogène, un groupe hydroxyle, ou un groupe alkyle dont le nombre d'atomes de carbone est de 1, 2, ou 3 qui peut être ramifié et, lorsqu'une pluralité de R¹⁶ sont présents, les R¹⁶ peuvent être des groupes identiques ou des groupes différents les uns des autres), dans lequel R¹¹ et R¹² présents dans la même molécule peuvent former un groupe carbonyle conjointement et R¹³ et R¹⁴ présents dans la même molécule peuvent former un groupe carbonyle conjointement, R¹⁵ représente un atome d'hydrogène, un atome d'halogène, ou un groupe alkyle dont le nombre d'atomes de carbone est de 1, 2, ou 3 qui peut être ramifié], dans lequel
dans une combinaison de X¹¹ à X¹³, l1+l2, m1+m2, n (l1, l2, m1, m2, et n représentent chacun indépendamment 0 ou 1), et de tirets doubles,
(a) X¹¹ est un atome de soufre, X¹² et X¹³ sont des atomes de carbone, l1+l2 vaut 2, m1+m2 vaut 2, n vaut 0, et les tirets doubles entre X¹¹ et X¹³ et entre X¹² et X¹³ sont des simples liaisons,
(b) X¹¹ est un atome de soufre, X¹² et X¹³ sont des atomes de carbone, l1+l2 vaut 1, m1+m2 vaut 1, n vaut 0, le tiret double entre X¹¹ et X¹³ est une simple liaison, et le tiret double entre X¹² et X¹³ est une double liaison,
(c) X¹¹ est un atome d'azote, X¹² et X¹³ sont des atomes de carbone, l1+l2 vaut 2, m1+m2 vaut 2, n vaut 1, et les tirets doubles entre X¹¹ et X¹³ et entre X¹² et X¹³ sont des simples liaisons,
(d) X¹¹ est un atome d'azote, X¹² et X¹³ sont des atomes de carbone, l1+l2 vaut 1, m1+m2 vaut 1, n vaut 1, le tiret double entre X¹¹ et X¹³ est une simple liaison, et le tiret double entre X¹² et X¹³ est une double liaison,
(e) X¹¹ et X¹² sont des atomes d'azote, X¹³ est un atome de carbone, l1+l2 vaut 1, m1+m2 vaut 1, n vaut 0, le tiret double entre X¹¹ et X¹³ est une double liaison, et le tiret double entre X¹² et X¹³ est une simple liaison, ou
(f) X¹¹, X¹², et X¹³ sont des atomes d'azote, l1+l2 vaut 0, m1+m2 vaut 0, n vaut 1, le tiret double entre X¹¹ et X¹³ est une simple liaison, et le tiret double entre X¹² et X¹³ est une double liaison, ou un sel de celui-ci.

3. Procédé selon l'une quelconque des revendications 1 à 2, dans lequel à l'étape consistant à amener l'anticorps anti-L-FABP et le composé ayant une structure partielle de NH₂-C=N- et une structure cyclique dans une molécule en contact avec un échantillon suspecté de contenir L-FABP, la concentration du composé est de 300 mmole/L à 500 mmole/L.

4. Procédé selon l'une quelconque des revendications 1 à 3, dans lequel l'anticorps anti-L-FABP est immobilisé sur un support insoluble, et le support insoluble est une particule de latex, ou une particule de colloïde métallique.

5. Procédé selon l'une quelconque des revendications 1 à 4, dans lequel l'anticorps anti-L-FABP est deux ou plus de deux anticorps monoclonaux ayant des sites de reconnaissance différents les uns des autres.

6. Procédé selon la revendication 5, dans lequel les deux ou plus de deux anticorps monoclonaux ayant des sites de reconnaissance différents l'un de l'autre sont respectivement immobilisés sur des particules de latex, et dans lequel L-FABP est détectée par un immuno-essai turbidimétrique sur latex.

7. Procédé selon l'une quelconque des revendications 1 à 6, dans lequel l'échantillon est de l'urine, du sang entier, du sérum, ou du plasma.

8. Procédé d'amélioration de la sensibilité de mesure dans un procédé de détection de L-FABP dans un échantillon avec un anticorps anti-L-FABP par un procédé de mesure d'agglutination de particules, comprenant l'étape consistant à : amener un anticorps anti-L-FABP et un composé ayant une structure partielle de NH₂-C=N- et une structure cyclique dans une molécule en contact avec un échantillon suspecté de contenir L-FABP, dans lequel
l'étape consistant à amener l'anticorps anti-L-FABP et le composé ayant une structure partielle de NH₂-C=N- et une structure cyclique dans une molécule en contact avec l'échantillon suspecté de contenir L-FABP est réalisée dans l'ordre dans lequel
une étape consistant à amener le composé en contact avec l'échantillon suspecté de contenir L-FABP est suivie par une étape consistant à amener le composé en contact avec l'anticorps anti-L-FABP ;
dans lequel le composé ayant une structure partielle de NH₂-C=N- et une structure cyclique dans une molécule est un ou deux ou plus de deux éléments choisis parmi un composé représenté par la formule (1) ou un sel ou ester de celui-ci et un composé représenté par la formule (2) ou un sel de celui-ci :
le composé de formule (1) 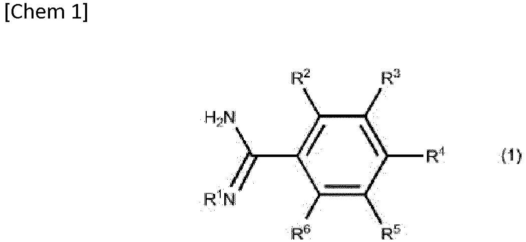 [dans la formule (1), R¹ est un atome d'hydrogène, un groupe hydroxyle, ou un groupe alkyle dont le nombre d'atomes de carbone est de 1, 2, ou 3 qui peut être ramifié, et R² à R⁶ représentent chacun indépendamment un atome d'hydrogène, un atome d'halogène, un groupe alkyle dont le nombre d'atomes de carbone est de 1, 2, ou 3 qui peut être ramifié, un groupe hydroxyle, un groupe carboxy, un groupe amino, ou -SR⁷ (R⁷ représente un atome d'hydrogène, un groupe hydroxyle, ou un groupe alkyle dont le nombre d'atomes de carbone est de 1, 2, ou 3 qui peut être ramifié et, lorsqu'une pluralité de R⁷ sont présents, les R⁷ peuvent être les mêmes groupes ou des groupes différents les uns des autres)] ou un sel ou ester de ceux-ci ; et
le composé de formule (2) 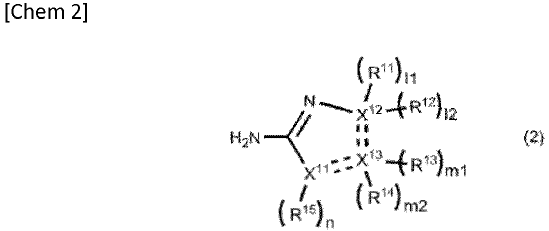
[dans la formule (2), R¹¹ à R¹⁴ représentent chacun indépendamment un atome d'hydrogène, un atome d'halogène, un groupe alkyle dont le nombre d'atomes de carbone est de 1, 2, ou 3 qui peut être ramifié, un groupe amino, un groupe phényle qui peut être substitué par un atome d'halogène, ou -SR¹⁶ (R¹⁶ représente un atome d'hydrogène, un groupe hydroxyle, ou un groupe alkyle dont le nombre d'atomes de carbone est de 1, 2, ou 3 qui peut être ramifié et, lorsqu'une pluralité de R¹⁶ sont présents, les R¹⁶ peuvent être des groupes identiques ou des groupes différents les uns des autres), dans lequel R¹¹ et R¹² présents dans la même molécule peuvent former un groupe carbonyle conjointement et R¹³ et R¹⁴ présents dans la même molécule peuvent former un groupe carbonyle conjointement, R¹⁵ représente un atome d'hydrogène, un atome d'halogène, ou un groupe alkyle dont le nombre d'atomes de carbone est de 1, 2, ou 3 qui peut être ramifié,
X¹¹ est un atome d'azote ou un atome de soufre,
X¹² et X¹³ sont chacun indépendamment un atome de carbone ou un atome d'azote, et
l1, l2, m1, m2, et n valent chacun indépendamment 0 ou 1,
un tiret double entre X¹¹ et X¹³ et un tiret double entre X¹² et X¹³ sont chacun indépendamment une simple liaison ou une double liaison, dans lequel
les valeurs de l1, l2, m1, m2, et n ainsi que les liaisons du tiret double entre X¹¹ et X¹³ et le tiret double entre X¹² et X¹³ indiquent des valeurs et des liaisons déterminées adéquatement selon les valences de X¹¹ à X¹³], ou sel de celui-ci.
